(19) Europäisches Patentamt
European Patent Office
Office européen des brevets

(11) Veröffentlichungsnummer: **0 591 785 A1**

(12) **EUROPÄISCHE PATENTANMELDUNG**

(21) Anmeldenummer: **93115451.2**

(22) Anmeldetag: **24.09.93**

(51) Int. Cl.5: **C07D 401/14**, C07D 409/14, A01N 47/38

(30) Priorität: **07.10.92 DE 4233717**

(43) Veröffentlichungstag der Anmeldung: **13.04.94 Patentblatt 94/15**

(84) Benannte Vertragsstaaten: **BE CH DE ES FR GB IT LI NL**

(71) Anmelder: **BAYER AG**

**D-51368 Leverkusen(DE)**

(72) Erfinder: **Fuchs, Rainer, Dr.**
**Am Rohm 107**
**D-42113 Wuppertal(DE)**
Erfinder: **Fischer, Reiner, Dr.**
**Nelly-Sachs-Strasse 23**
**D-40789 Monheim(DE)**
Erfinder: **Erdelen, Christoph, Dr.**
**Unterbüscherhof 22**
**D-42799 Leichlingen(DE)**
Erfinder: **Stendel, Wilhelm, Dr.**
**In den Birken 56**
**D-42113 Wuppertal(DE)**

(54) **Substituierte 3,4-Hetarylpyrazoline, Verfahren zu ihrer Herstellung und ihre als Schädlingsbekämpfungsmittel.**

(57) Die vorliegende Erfindung betrifft neue substituierte 3,4-Hetaryl-pyrazoline der allgemeinen Formel (I)

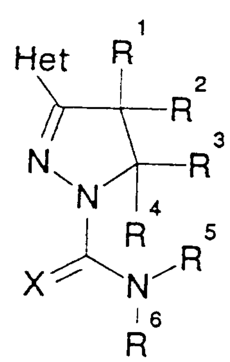

(I)

in welcher

$R^1$ für einen ungesättigten fünf- oder sechsgliedrigen, 1 bis 4 Stickstoffatome enthaltenden, gegebenenfalls substituierten und gegebenenfalls benzokondensierten Heterocyclus oder für einen gegebenenfalls substituierten Heterocyclus aus der Reihe

EP 0 591 785 A1

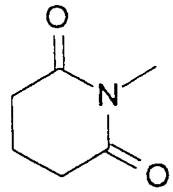 , 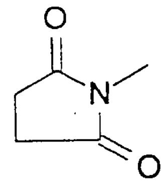

, 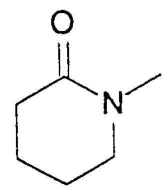 , 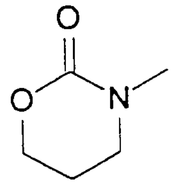 , 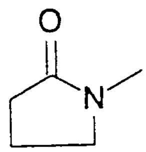 , 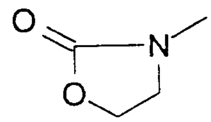

steht,

Verfahren zu ihrer Herstellung und ihre Verwendung als Schädlingsbekämpfungsmittel.

Die Erfindung betrifft neue substituierte 3,4-Hetaryl-pyrazoline, mehrere Verfahren zu ihrer Herstellung und ihre Verwendung als Schädlingsbekämpfungsmittel.

Es ist bekannt, daß bestimmte substituierte Pyrazolin-Derivate eine gute Wirksamkeit gegen tierische Schädlinge besitzen.

Siehe dazu z.B. DE-A 2 700 258, US-A 4 174 393, DE-A 2 529 689, US-A 4 070 365 und EP 0 466 408, DE-A 4 001 931, DE-A 4 117 076, US-P 5 068 241.

Die Wirkungshöhe bzw. Wirkungsdauer dieser vorbekannten Verbindungen ist jedoch, insbesondere gegen bestimmte Organismen oder bei niedrigen Anwendungskonzentrationen, nicht in allen Anwendungsgebieten völlig zufriedenstellend.

Es wurden neue substituierte 3,4-Hetaryl-pyrazoline der allgemeinen Formel (I)

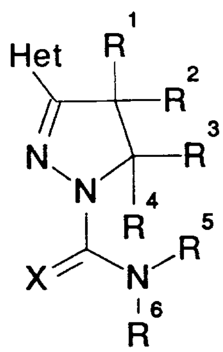

(I)

in welcher

$R^1$ für einen ungesättigten fünf- oder sechsgliedrigen, 1 bis 4 Stickstoffatome enthaltenden, gegebenenfalls substituierten und gegebenenfalls benzokondensierten Heterocyclus oder für einen gegebenenfalls substituierten Heterocyclus aus der Reihe

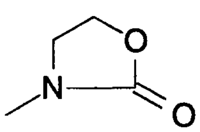 , 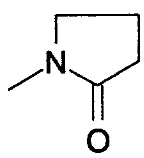 , 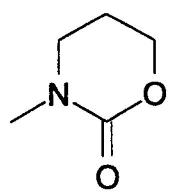 , 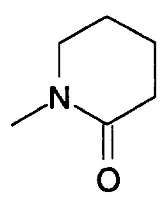 ,

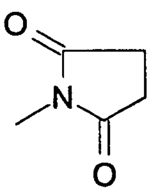 , 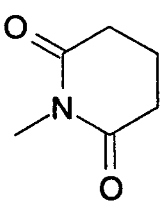

steht

$R^2$ für Wasserstoff, Alkyl, gegebenenfalls substituiertes Cycloalkyl, Halogenalkyl, Halogenalkylthio oder Alkoxycarbonyl steht,

$R^3$ für Wasserstoff oder Alkyl steht,

$R^4$ für Wasserstoff oder Alkyl steht,

$R^5$ für Wasserstoff, Alkyl, Phenyl oder Alkylthio steht,

$R^6$ für gegebenenfalls substituiertes Alkyl, gegebenenfalls substituiertes Cycloalkyl oder für den Rest

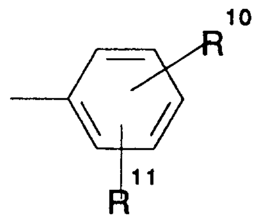

steht,
worin $R^{10}$ und $R^{11}$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

X für Sauerstoff oder Schwefel steht und

Het für einen gegebenenfalls substituierten und/oder gegebenenfalls anellierten Heterocyclus steht,

gefunden.

Weiterhin wurde gefunden, daß man die neuen substituierten 3,4-Hetaryl-pyrazoline der allgemeinem Formel (I)
in welcher

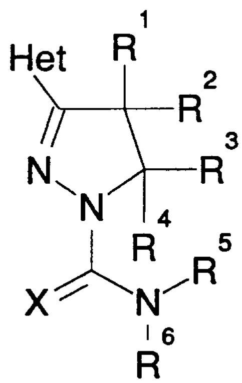

(I)

$R^1$ für einen ungesättigten fünf- oder sechsgliedrigen, 1 bis 4 Stickstoffatome enthaltenden, gegebenenfalls substituierten und gegebenenfalls benzokondensierten Heterocyclus oder für einen gegebenenfalls substituierten Heterocyclus aus der Reihe

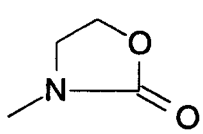 , 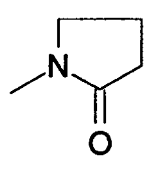 , 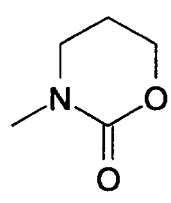 , 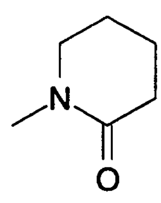 ,

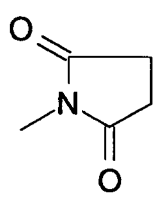 , 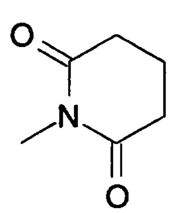

steht

$R^2$ für Wasserstoff, Alkyl, gegebenenfalls substituiertes Cycloalkyl, Halogenalkyl, Halogenalkylthio oder Alkoxycarbonyl steht,
$R^3$ für Wasserstoff oder Alkyl steht,
$R^4$ für Wasserstoff oder Alkyl steht,
$R^5$ für Wasserstoff, Alkyl, Phenyl oder Alkylthio steht,
$R^6$ für gegebenenfalls substituiertes Alkyl, gegebenenfalls substituiertes Cycloalkyl oder für den Rest

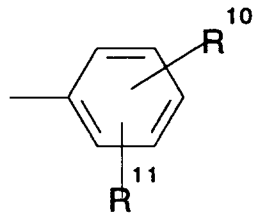

steht,
worin $R^{10}$ und $R^{11}$ gleich oder verschieden sein können und für Wasserstoff, Haloen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes Arylthio, Alkenyloxy, Alkinyl, Alkylhionyl, Alkylsulfonyl, Halogenalkylthionyl, Halogenalkylsulfonyl, Halogenlkxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,
X für Sauerstoff oder Schwefel steht und
Het für einen gegebenenfalls substituierten und/oder gegebenenfalls anellierten Heteroyclus steht,

erhält, wenn man

(A) zum Erhalt von substituierte 3,4-Hetaryl-pyrazolinen der Formel (I), in welcher $R^5$ für Wasserstoff steht, Pyrazolinderivate der Formel (II)

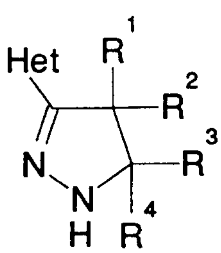

(II)

in welcher
$R^1$, $R^2$, $R^3$, $R^4$ und Het die oben angegebene Bedeutung besitzen, mit Isocyanaten bzw. Isothiocyanaten der Formel (III)

$$X = C = N\text{-}R^6 \qquad \text{(III)}$$

in welcher
X und $R^6$ die oben angegebene Bedeutung haben, gegebenenfalls in Gegenwart von Basen umsetzt oder wenn man

(B) Pyrazolinderivate der Formel (II)

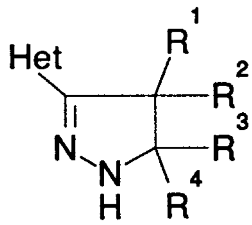

(II)

in welcher

$R^1$, $R^2$, $R^3$, $R^4$ und Het die oben angegebene Bedeutung haben, mit Verbindungen der Formel (IV)

$R^6$-NH-CO-Cl (IV)

in welcher

$R^6$ die oben angegebene Bedeutung hat,

gegebenenfalls in Gegenwart eines Lösungsmittels und gegebenenfalls in Gegenwart von Basen umsetzt.

Schließlich wurde gefunden, daß die neuen substituierten 3,4-Hetaryl-pyrazolineder allgemeinen Formel (I) eine sehr gute Wirkung gegen Schädlinge und insbesondere eine sehr gute insektizide und akarizide Wirksamkeit besitzen.

Überraschenderweise zeigen die erfindungsgemäßen substituierten 3,4-Hetaryl-pyrazoline eine erheblich bessere insektizide Wirksamkeit gegen pflanzenschädigende und warmblüterparasitierende Insekten und Spinnentiere als aus dem Stand der Technik bekannte chemisch und wirkungsmäßig naheliegende Verbindungen.

Die erfindungsgemäßen substituierten 3,4-Hetaryl-pyrazoline sind durch die Formel (I) allgemein definiert. Bevorzugt sind Verbindungen der Formel (I), bei welchen

$R^1$ für einen gegebenenfalls substituierten Pyrrolyl-, Pyrazolyl-, Imidazolyl-, 1,2,3-Triazolyl-, 1,2,4-Triazolyl-, Tetrazolyl-, Pyridinyl-, Pyridonyl-, Pyrimidinyl-, Pyrimidonyl-, Pyrazinyl-, Pyridazinyl- oder Triazinyl-Rest steht, der weiterhin für einen gegebenenfalls substituierten und über Stickstoff gebunenen Azolinon-, Azolinthion- oder Azoliniminorest aus der Reihe

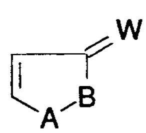 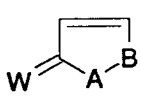 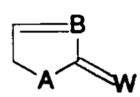 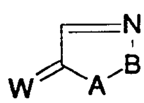 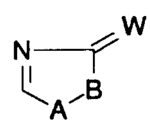 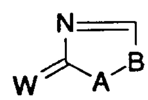

($R^1$-a) ($R^1$-b) ($R^1$-c) ($R^1$-d) ($R^1$-e) ($R^1$-f)

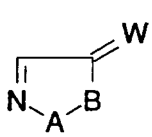 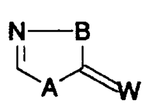 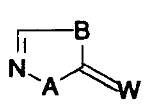 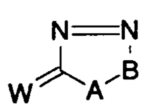 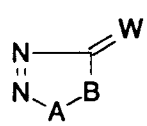 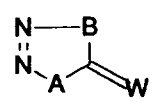

($R^1$-g) ($R^1$-h) ($R^1$-i) ($R^1$-k) ($R^1$-l) ($R^1$-m)

worin

eine der Gruppen A oder B für Stickstoff steht und jeweils die andere (A oder B) für Sauerstoff, Schwefel oder für die Gruppe -NAlkyl($C_1$-$C_4$-), für eine Methylengruppierung $-CH_2-$ oder eine -CH-Gruppe steht,

W für Sauerstoff, Schwefel oder für die Gruppe -NAlkyl($C_1$-$C_4$-) steht, oder weiterhin für

einen gegebenenfalls substituierten Heterocyclus aus der Reihe

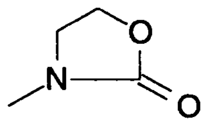, 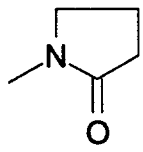, 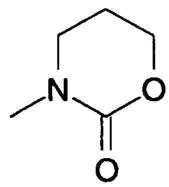, 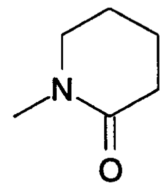,

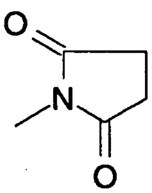, 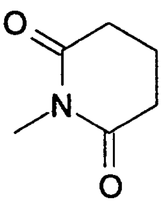

steht worin für die oben aufgeführten Heterocyclen jeweils die folgenden Subtuenten infrage kommen:

Halogen, Cyano, Nitro, Hydroxy, Amino, Alkyl($C_1$-$C_6$), Alkoxy($C_1$-$C_6$), Alkyl($C_1$-$C_6$)thio, Halogenalkyl($C_1$-$C_4$), Halogenalkoxy($C_1$-$C_4$), Halogenalkyl($C_1$-$C_4$)thio, Alkyl($C_1$-$C_6$)amino, Dialkyl($C_1$-$C_6$)amino, Dihalogenalkyl($C_1$-$C_4$)amino, Alkoxy($C_1$-$C_6$)carbonyl und gegebenenfalls durch Halogen, Alkyl($C_1$-$C_4$), Alkoxy($C_1$-$C_4$), Halogenalkyl($C_1$-$C_4$), Halogenalkoxy($C_1$-$C_4$) oder Halogenalkyl($C_1$-$C_4$)thio substituiertes Phenyl,

$R^2$ für Wasserstoff, Alkyl($C_1$-$C_6$), gegebenenfalls durch Halogen, Halogenalkyl($C_1$-$C_4$) substituiertes Cycloalkyl($C_3$-$C_7$); Halogenalkyl($C_1$-$C_4$)thio oder Alkoxy($C_1$-$C_6$)carbonyl steht,

$R^3$ für Wasserstoff oder Alkyl($C_1$-$C_6$) steht,

$R^4$ für Wasserstoff oder Alkyl($C_1$-$C_6$) steht,

$R^5$ für Wasserstoff, Alkyl($C_1$-$C_6$), Phenyl oder Alkyl($C_1$-$C_4$)thio steht,

$R^6$ für gegebenenfalls durch Halogen, Halogenalkyl($C_1$-$C_4$), Halogenalkoxy($C_1$-$C_4$) substituiertes Alkyl($C_1$-$C_6$),

für gegebenenfalls durch Halogen, Halogenalkyl($C_1$-$C_4$), Halogenalkoxy($C_1$-$C_4$) substituiertes Cycloalkyl($C_3$-$C_7$) oder für den Rest

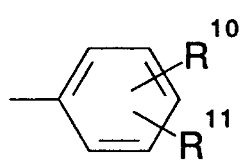

steht,

wobei

$R^{10}$ und $R^{11}$ gleich oder verschieden sein können und für Wasserstoff, Halogen, Alkyl($C_1$-$C_6$), Nitro, Cyano, Halogenalkyl($C_1$-$C_4$), Alkoxy($C_1$-$C_6$), Halogenalkoxy($C_1$-$C_4$), Alkyl($C_1$-$C_4$)thio, Halogenalkyl($C_1$-$C_4$)thio, gegebenenfalls durch Halogen, Halogenalkyl($C_1$-$C_4$), Alkoxy($C_1$-$C_4$), Alkyl($C_1$-$C_4$) substituiertes Phenoxy oder Phenylthio, gegebenenfalls durch Halogen, Alkoxy($C_1$-$C_4$), Halogenalkyl($C_1$-$C_4$) substituiertes Mono- oder Dialkylamino mit jeweils 1 bis 6 Kohlenstoffatomen im Alkylrest, gegebenenfalls durch Alkyl($C_1$-$C_4$), Alkoxy($C_1$-$C_4$), Halogen, Alkyl($C_1$-$C_4$)thio substituiertes Cycloalkyl($C_3$-$C_7$), Alkoxy($C_1$-$C_4$)-carbonyl, Alkenyl($C_2$-$C_6$)oxy, Alkinyl($C_2$-$C_6$), Alkyl($C_1$-$C_4$)thionyl, Alkyl($C_1$-$C_4$)sulfonyl, Halogenalkyl($C_1$-$C_4$)thionyl, Halogenalkyl($C_1$-$C_4$)sulfonyl, Halogenalkoxy($C_1$-$C_4$)-carbonyl stehen oder wobei

$R^{10}$ und $R^{11}$ zusammen für einen der folgenden bivalenten Reste stehen

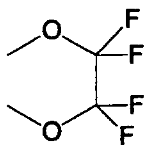 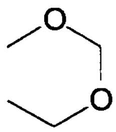 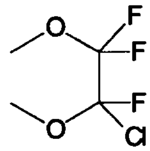 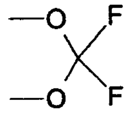

X für Sauerstoff oder Schwefel steht und

Het für einen unsubstituierten oder einfach bis mehrfach, gleich oder verschieden substituierten, gegebenenfalls benzoanellierten heteroaromatischen 5-oder 6-gliedrigen Ring, der gleiche oder verschiedene, ein oder mehrere Heteroatome wie Sauerstoff, Schwefel und Stickstoff enthält, wobei als Substituenten infrage kommen
Alkyl, Halogen, Halogenalkyl, Alkoxy, Alkylthio, Halogenalkoxy, Halogenalkylthio, Alkoxycarbonyl, Halogenalkoxycarbonyl, gegebenenfalls substituiertes Aryloxy, gegebenenfalls substituiertes Arylthio, Alkenyloxy, Alkinyl, Alkylthionyl, Alkylsulfonyl, Halogenalkylthionyl, Halogenalkylsulfonyl, Nitro, Cyano oder wobei zwei benachbarte Positionen durch einen durch Halogen substituierten 3,4-Methylendioxyl- oder 3,4-Ethylendioxylrest miteinander verbunden sind.

Besonders bevorzugt sind die Verbindungen der Formel (I), bei welchen

$R^1$ für einen gegebenenfalls substituierten Pyrrolyl-, Pyrazolyl-, Imidazolyl-, 1,2,3-Triazolyl-, Tetrazolyl-, Pyridinyl-, Pyridonyl-, Pyrimidinyl-, Pyrimidonyl-, Pyrazinyl-, Pyridazinyl-, 1,2,4-Triazolyl- oder Triazinyl-Rest steht oder

$R^1$ weiterhin für einen substituierten oder einfach oder zweifach, gleich oder verschieden substituierten und über Stickstoff gebundenen Azolinon, Azolinthion-oder Azoliniminrest aus der Reihe

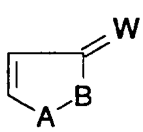 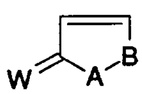 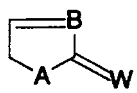 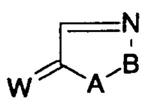 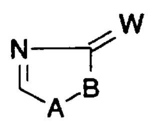 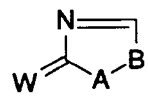

($R^1$-a) ($R^1$-b) ($R^1$-c) ($R^1$-d) ($R^1$-e) ($R^1$-f)

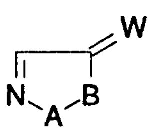 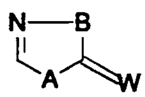 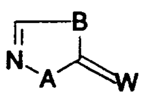 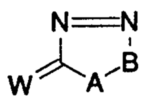 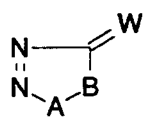 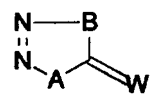

($R^1$-g) ($R^1$-h) ($R^1$-i) ($R^1$-k) ($R^1$-l) ($R^1$-m)

worin

eine der Gruppen A oder B für Stickstoff steht und jeweils die andere (A oder B) für Sauerstoff, Schwefel oder für die Gruppe - NAlkyl($C_1$-$C_4$-), für eine Methylengruppierung $-CH_2-$ oder eine CH-Gruppe steht,

W für Sauerstoff, Schwefel oder für die Gruppe - NAlkyl($C_1$-$C_4$-) steht, oder

$R^1$ weiterhin für einen unsubstituierten oder einfach bis dreifach substituierten Heterocyclus aus der Reihe

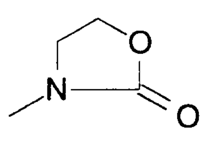 , 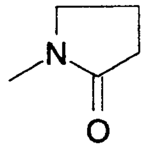 , 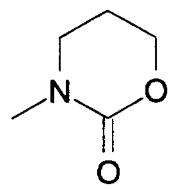 , 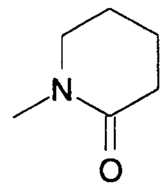 ,

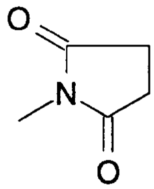 , 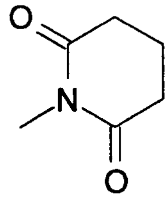

steht
worin für die oben aufgeführten Heterocyclen jeweils die folgenden Substituenten infrage kommen:
Fluor, Chlor, Brom, Iod, Cyano, Nitro, Hydroxy, Amino, Alkyl($C_1$-$C_4$), Alkoxy($C_1$-$C_4$), Alkyl($C_1$-$C_4$)thio, Halogenalkyl($C_1$-$C_2$), Halogenalkoxy($C_1$-$C_2$), Halogenalkyl($C_1$-$C_2$)thio, Alkyl($C_1$-$C_3$)amino, Dialkyl($C_1$-$C_3$)amino, Dihalogenalkyl($C_1$-$C_2$)amino, Alkoxy($C_1$-$C_4$)carbonyl und gegebenenfalls durch Fluor, Chlor, Brom, Methyl, Ethyl, Methoxy, Ethoxy, Halogenalkyl-($C_1$-$C_2$), Halogenalkoxy($C_1$-$C_2$) oder Halogenalkyl($C_1$-$C_2$)thio substituiertes Phenyl,

$R^2$ für Wasserstoff, Alkyl($C_1$-$C_4$), gegebenenfalls durch Fluor, Chlor, Brom, Halogenalkyl($C_1$-$C_3$) substituiertes Cycloalkyl($C_3$-$C_6$); Halogenalkyl($C_1$-$C_3$)thio oder Alkoxy($C_1$-$C_4$)carbonyl steht,

$R^3$ für Wasserstoff oder Alkyl($C_1$-$C_4$) steht,

$R^4$ für Wasserstoff oder Alkyl($C_1$-$C_4$) steht,

$R^5$ für Wasserstoff, Alkyl($C_1$-$C_4$), Phenyl oder Alkyl($C_1$-$C_3$)thio steht,

$R^6$ für gegebenenfalls durch Fluor, Chlor, Brom, Halogenalkyl($C_1$-$C_3$), Halogenalkoxy($C_1$-$C_3$) substituiertes Alkyl($C_1$-$C_4$), für gegebenenfalls durch Fluor, Chlor, Brom, Halogenalkyl($C_1$-$C_3$), Halogenalkoxy($C_1$-$C_3$) substituiertes Cycloalkyl($C_3$-$C_6$) oder für den Rest

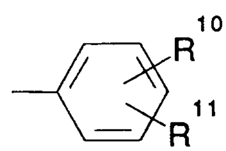

steht,
wobei

$R^{10}$ und $R^{11}$ gleich oder verschieden sein können und für Wasserstoff, Fluor, Chlor, Brom, Iod, Alkyl($C_1$-$C_4$), Nitro, Cyano, Halogenalkyl($C_1$-$C_3$), Alkoxy($C_1$-$C_4$), Halogenalkoxy($C_1$-$C_3$), Alkyl($C_1$-$C_3$)thio, Halogenalkyl($C_1$-$C_3$)thio, gegebenenfalls durch Fluor, Chlor, Brom, Halogenalkyl($C_1$-$C_3$),Alkoxy($C_1$-$C_3$), Alkyl($C_1$-$C_3$) substituiertes Phenoxy, gegebenenfalls durch Fluor, Chlor, Brom, Alkoxy($C_1$-$C_3$), Halogenalkyl($C_1$-$C_3$) substituiertes Mono- oder Dialkylamino mit jeweils 1 bis 4 Kohlenstoffatomen im Alkylrest, gegebenenfalls durch Alkyl($C_1$-$C_3$), Alkoxy($C_1$-$C_3$), Fluor, Chlor, Brom, Alkyl($C_1$-$C_3$)thio substituiertes Cycloalkyl($C_3$-$C_6$) stehen oder wobei

$R^{10}$ und $R^{11}$ zusammen für einen der folgenden bivalenten Reste stehen

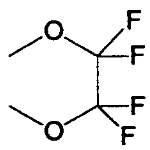 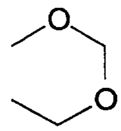 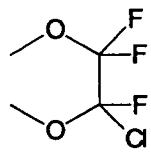 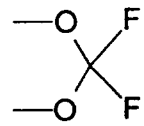

X für Sauerstoff oder Schwefel steht und

Het für einen unsubstituierten oder einfach bis dreifach, gleich oder verschieden substituierten heteroaromatischen 5- oder 6-gliedrigen Ring steht, der gleiche oder verschiedene, ein oder mehrere Heteroatome wie Sauerstoff, Schwefel oder Stickstoff enthält, wobei als Substituenten genannt seien:
Alkyl($C_1$-$C_4$), Fluor, Chlor, Brom, Halogenalkyl($C_1$-$C_4$), Alkoxy($C_1$-$C_4$), Alkyl($C_1$-$C_4$)thio, Halogenalkoxy($C_1$-$C_3$), Halogenalkyl($C_1$-$C_3$)thio, Alkoxy-($C_1$-$C_3$)carbonyl, gegebenenfalls durch Fluor, Chlor, Brom, Alkyl($C_1$-$C_3$)-,Alkoxy($C_1$-$C_3$), Halogenalkyl($C_1$-$C_3$) substituiertes Phenoxy oder Phenylthio, Alkenyl($C_2$-$C_4$)oxy, Alkinyl($C_2$-$C_4$), Alkyl($C_1$-$C_3$)thionyl, Alkyl($C_1$-$C_3$)-sulfonyl, Halogenalkyl($C_1$-$C_3$)thionyl, Halogenalkyl($C_1$-$C_3$)sulfonyl, Nitro, Cyano stehen oder
wobei zwei benachbarte Positionen durch einen durch Fluor und/oder Chlor substituierten 3,4-Methylendioxy- oder 3,4-Ethylendioxyrest miteinander verbunden sind.

Insbesondere bevorzugt sind Verbindungen der Formel (I) bei welchen

$R^1$ für einen gegebenenfalls substituierten Pyrrolyl-, Pyrazolyl-, Imidazolyl-, 1,2,3-Triazolyl-, 1,2,4-Triazolyl-, Tetrazolyl-, Pyridinyl-, Pyridonyl-, Pyrimidinyl-, Pyrimidonyl-, Pyrazinyl-, Pyridazinyl-oder Triazinyl-Rest steht oder

$R^1$ weiterhin für einen unsubstituierten oder einfach oder zweifach, gleich oder verschieden substituierten und über Stickstoff gebundenen Azolinon, Azolinthion-oder Azoliniminrest aus der Reihe

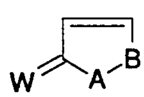
($R^1$-b)

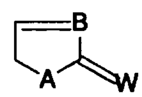
($R^1$-c)

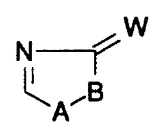
($R^1$-e)

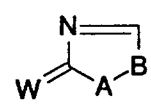
($R^1$-f)

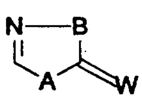
($R^1$-h)

oder

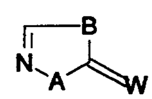
($R^1$-i)

worin

eine der Gruppen A oder B für Stickstoff steht und jeweils die andere (A oder B) für Sauerstoff, Schwefel oder für die Gruppe - NAlkyl($C_1$-$C_4$-), für eine Methylengruppierung -$CH_2$- oder eine - CH-Gruppe steht,

W für Sauerstoff oder Schwefel steht, oder

$R^1$ weiterhin für einen unsubstituierten oder einfach bis dreifach substituierten Heterocyclus aus der Reihe

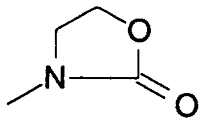, 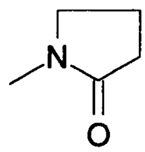, 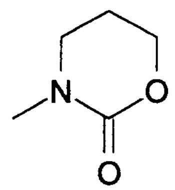, 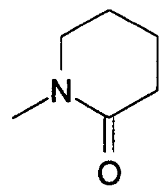,

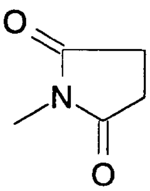, 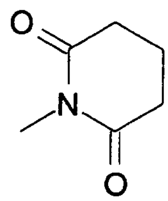

steht wobei für die oben genannten Heterocyclen jeweils die folgenden Substituenten infrage kommen:
Fluor, Chlor, Brom, Iod, Cyano, Nitro, Hydroxy, Amino, Methyl, Ethyl, n-Propyl, i-Propyl, t-Butyl, Methoxy, Ethoxy, Methylthio, Ethylthio, Halogenalkyl($C_1$-$C_2$), Halogenalkoxy($C_1$-$C_2$), Halogenalkyl($C_1$-$C_2$)thio mit jeweils 1 bis 5 Fluor- und/oder Chloratomen, Methylamino, Dimethylamino, Methoxycarbonyl und gegebenenfalls durch Fluor, Chlor, Brom, Methyl, Ethyl, Methoxy, Ethoxy und Halogenalkoxy($C_1$-$C_2$) oder Halogenalkyl($C_1$-$C_2$)thio mit jeweils 1 bis 5 Fluor- und/oder Chloratomen substituiertes Phenyl,

$R^2$ für Wasserstoff, Methyl, Ethyl, n-Propyl, i-Propyl oder Alkoxy($C_1$-$C_2$)-carbonyl steht,

$R^3$ für Wasserstoff, Methyl, Ethyl, n-Propyl oder i-Propyl steht,

$R^4$ für Wasserstoff, Methyl, Ethyl oder n-Propyl steht,

$R^5$ für Wasserstoff, Methyl, Ethyl, n-Propyl, i-Propyl, Phenyl oder Alkyl($C_1$-$C_2$)- thio steht,

$R^6$ für gegebenenfalls durch Fluor, Chlor, Halogenalkyl($C_1$-$C_3$), Halogenalkoxy($C_1$-$C_3$) substituiertes Methyl, Ethyl, n-Propyl oder i-Propyl für gegebenenfalls durch Fluor, Chlor, Halogenalkyl($C_1$-$C_3$)-,Halogenalkoxy($C_1$-$C_3$) substituiertes Cycloalkyl ($C_3$-$C_6$) oder für den Rest

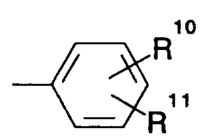

steht,
wobei

$R^{10}$ und $R^{11}$ gleich oder verschieden sein können und für Wasserstoff, Fluor, Chlor, Brom, Iod, Methyl, Ethyl, n-Propyl, i-Propyl, tert-Butyl, Nitro, Cyano, Halogenalkyl($C_1$-$C_3$), Alkoxy($C_1$-$C_3$), Halogenalkoxy($C_1$-$C_3$), Alkyl($C_1$-$C_3$)-thio, Halogenalkyl($C_1$-$C_3$)thio, gegebenenfalls durch Fluor, Chlor, Halogenalkyl($C_1$-$C_3$), Methoxy, Ethoxy, Methyl, Ethyl substituiertes Phenoxy, gegebenenfalls durch Fluor, Chlor, Methoxy, Ethoxy, Halogenalkyl-($C_1$-$C_3$) substituiertes Mono-oder Dialkylamino mit jeweils 1 bis 3 Kohlenstoffatomen im Alkylrest, gegebenenfalls durch Methyl, Ethyl, Methoxy, Ethoxy, Fluor, Chlor, Alkyl($C_1$-$C_3$)thio substituiertes Cycloalkyl($C_3$-$C_6$) stehen oder wobei

$R^{10}$ und $R^{11}$ zusammen für einen der folgenden bivalenten Reste stehen

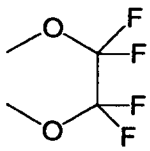 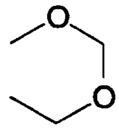 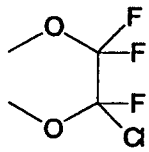 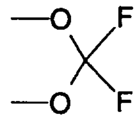

X für Sauerstoff oder Schwefel steht und

Het für einen jeweils unsubstituierten oder einfach bis dreifach, gleich oder verschieden substituierten Pyridyl-, Pyrazinyl-, Thiazolyl-, Pyrimidyl-, Pyridazinyl, Thienyl-, Furyl-, Oxazolyl- oder Pyrrolyl-Rest steht, wobei als Substituenten genannt seien:

Methyl, Ethyl, n-Propyl, i-Propyl, tert-Butyl, Fluor, Chlor,Brom, Iod, Halogenalkyl($C_1$-$C_3$), Methoxy, Ethoxy, n-Propyloxy, i-Propyloxy, Alkyl-($C_1$-$C_3$)thio, Halogenalkoxy($C_1$-$C_3$), Halogenalkyl($C_1$-$C_3$)thio, Alkoxy($C_1$-$C_3$)carbonyl, gegebenenfalls durch Fluor, Chlor, Brom, Methyl, Ethyl, Methoxy, Ethoxy, Halogenalkyl($C_1$-$C_3$) substituiertes Phenoxy oder Phenylthio, Alkenyl($C_3$-$C_4$)oxy, Alkinyl($C_2$-$C_4$), Alkyl($C_1$-$C_3$)thionyl, Alkyl($C_1$-$C_3$)sulfonyl, Halogenalkyl($C_1$-$C_3$)thionyl, Halogenalkyl($C_1$-$C_3$)sulfonyl, Nitro, Cyano stehen oder

wobei zwei benachbarte Positionen durch einen durch Fluor und/oder Chlor substituierten 3,4-Methylendioxy- oder 3,4-Ethylendioxyrest miteinander verbunden sind.

Eine Gruppe von außerordentlich bevorzugten Verbindungen der Formel (I) sind diejenigen, bei welchen

$R^1$ für einen gegebenenfalls substituierten Pyrrolyl-, Pyrazolyl-, Imidazolyl-, 1,2,3-Triazolyl-, 1,2,4-Triazolyl-, Tetrazolyl-, Pyridinyl-, Pyridonyl-, Pyrimidinyl-, Pyrazinyl-, Pyridazinyl-oder Triazinyl-Rest steht,

$R^1$ weiterhin für einen unsubstituierten oder einfach oder zweifach, gleich oder verschieden substituierten und über Stickstoff gebundenen Azolinon, Azolinthion-oder Azoliniminrest aus der Reihe

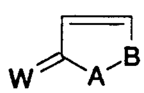

($R^1$-b)

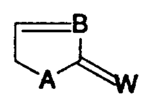

($R^1$-c)

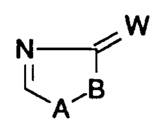

($R^1$-e)

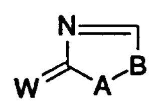

($R^1$-f)

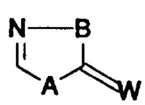

($R^1$-h)

oder

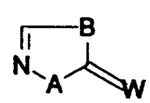

($R^1$-i)

worin

eine der Gruppen A oder B für Stickstoff steht und jeweils die andere (A oder B) für Sauerstoff, Schwefel oder für die Gruppe - NAlkyl($C_1$-$C_4$-), für eine Methylengruppierung - $CH_2$- oder eine -CH-Gruppe steht,

W für Sauerstoff oder Schwefel steht,

oder

wobei für die oben genannten Heterocyclen jeweils die folgenden Substituenten infrage kommen:

Fluor, Chlor, Brom, Iod, Cyano, Nitro, Hydroxy, Amino, Methyl, Ethyl, n-Propyl, i-Propyl, t-Butyl, Methoxy, Ethoxy, Methylthio, Ethylthio,

Halogenalkyl($C_1$-$C_2$), Halogenalkoxy($C_1$-$C_2$), Halogenalkyl($C_1$-$C_2$)thio mit jeweils 1 bis 5 Fluor- und/oder Chloratomen, Methylamino, Dimethylamino, Methoxycarbonyl und gegebenenfalls durch Fluor, Chlor, Brom, Methyl, Ethyl, Methoxy, Ethoxy und Halogenalkoxy($C_1$-$C_2$) oder Halogenalkyl($C_1$-$C_2$)thio mit jeweils 1 bis 5 Fluor- und/oder Chloratomen substituiertes Phenyl,
und

$R^2$, $R^3$, $R^4$, $R^5$, $R^6$, X und Het die oben angegebene Bedeutung haben.

Die Substituentenbedeutung Halogenalkyl in den Resten Halogenalkyl, Halogenalkoxy, Halogenalkylthio, Halogenalkylthionyl und Halogenalkylsulfonyl enthält vorzugsweise 1 bis 4, insbesondere 1 oder 2 Kohlenstoffatome und vorzugsweise 1 bis 5, insbesondere 1 bis 3 gleiche oder verschiedene Halogenatome, wobei als Halogenatome vorzugsweise Fluor, Chlor und Brom, insbesondere Fluor und Chlor stehen. Beispielhaft seien Trifluormethyl, Chlordifluormethyl, Brommethyl, 2,2,2-Trifluorethyl und Pentafluorethyl genannt.

Im einzelnen seien beispielhaft, aber nicht einschränkend außer den bei den Herstellungsbeispielen genannten Verbindungen die folgenden in Tabelle 1 aufgeführten substituierten 3,4-Hetaryl-pyrazoline der Formel (I) genannt:

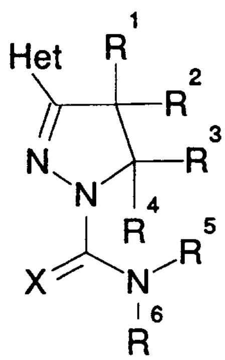

(I)

Tabelle 1:

| Het | $R^1$ | $R^2$ | $R^3$ | $R^4$ | $R^5$ | $R^6$ | X |
|---|---|---|---|---|---|---|---|
| 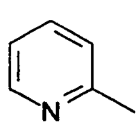 | 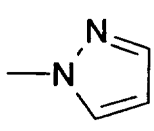 | H | H | H | H | 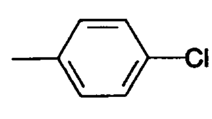 | O |
| 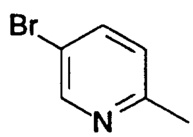 | 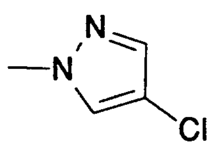 | H | H | H | H | 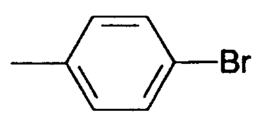 | O |

Tabelle 1:(Fortsetzung)

| Het | $R^1$ | $R^2$ | $R^3$ | $R^4$ | $R^5$ | $R^6$ | X |
|---|---|---|---|---|---|---|---|
| 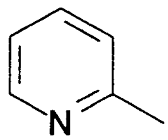 | 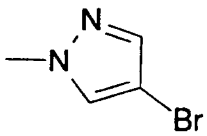 | H | H | H | H | 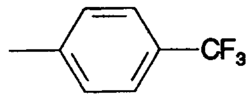 | O |
| 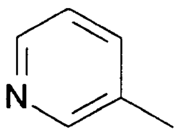 | 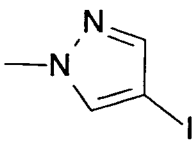 | H | H | H | H | 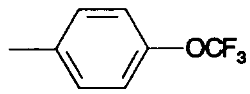 | O |
| 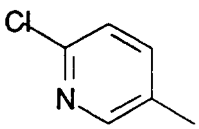 | 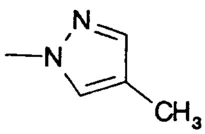 | H | H | H | H | 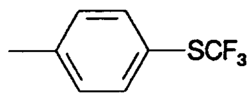 | O |
| 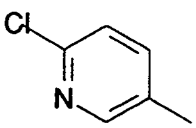 | 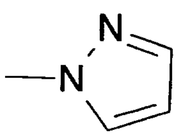 | H | H | H | H | 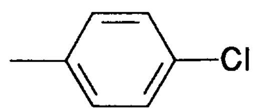 | O |
| 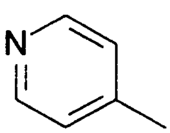 | 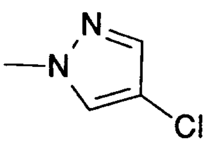 | $CH_3$ | H | H | H | 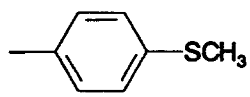 | O |

Tabelle 1 (Fortsetzung)

| Het | $R^1$ | $R^2$ | $R^3$ | $R^4$ | $R^5$ | $R^6$ | X |
|---|---|---|---|---|---|---|---|
| 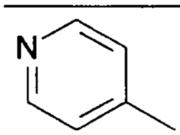 | 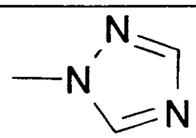 | H | H | H | H | 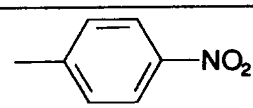 | O |
| 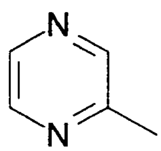 | 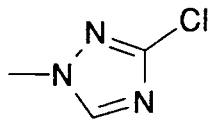 | 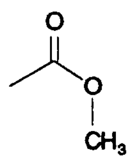 | H | H | H | 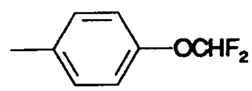 | O |
| 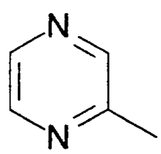 | 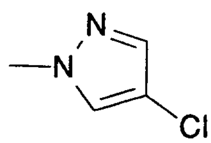 | H | H | H | H | 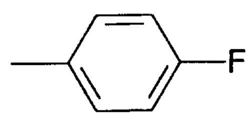 | O |
| 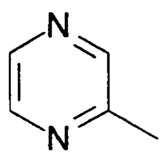 | 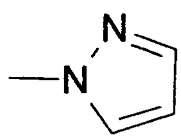 | H | H | H | H | 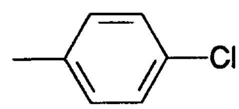 | O |
| 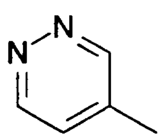 | 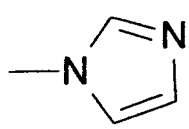 | H | H | H | H | 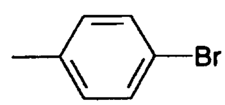 | O |
| 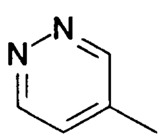 | 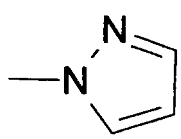 | H | H | H | H | 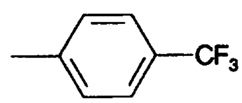 | O |

Tabelle 1: (Fortsetzung)

| Het | $R^1$ | $R^2$ | $R^3$ | $R^4$ | $R^5$ | $R^6$ | X |
|---|---|---|---|---|---|---|---|
| 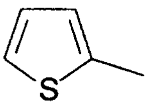 | 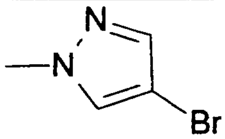 | H | H | H | H | 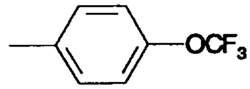 | O |
| 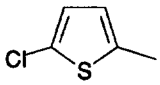 | 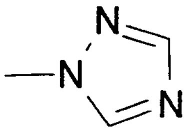 | H | H | H | H | 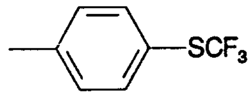 | O |
| 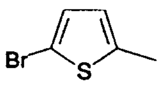 | 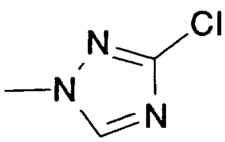 | H | H | H | H | 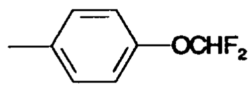 | O |
| 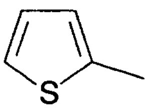 | 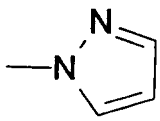 | H | H | H | H | 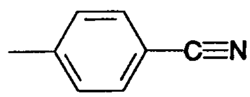 | O |
| 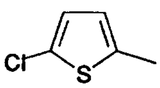 | 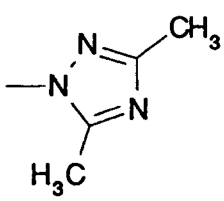 | H | H | H | H | 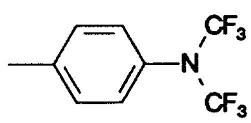 | O |
| 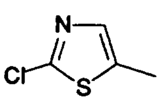 | 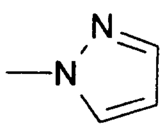 | H | H | H | H | 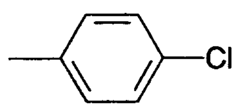 | S |
| 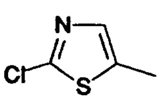 | 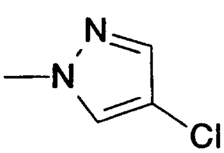 | H | H | H | H | 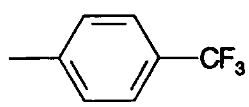 | O |

Tabelle 1: (Fortsetzung)

| Het | $R^1$ | $R^2$ | $R^3$ | $R^4$ | $R^5$ | $R^6$ | X |
|---|---|---|---|---|---|---|---|
| 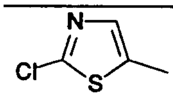 | 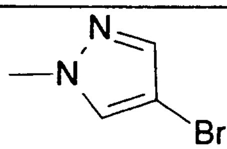 | H | H | H | H | 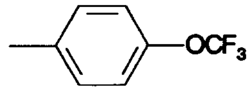 | O |
| 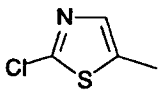 | 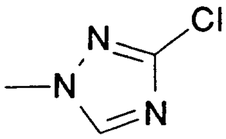 | H | H | H | H | 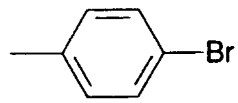 | O |
| 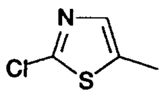 | 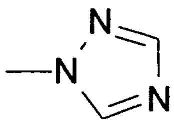 | H | H | H | H | 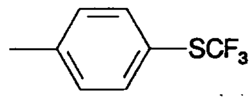 | O |
| 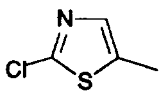 | 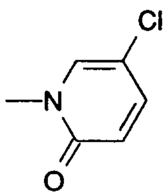 | H | H | H | H | 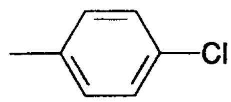 | O |
| 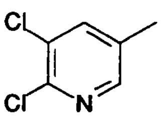 | 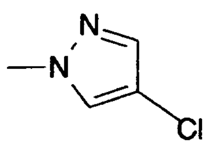 | H | H | H | H | 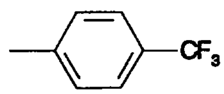 | O |
| 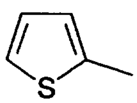 | 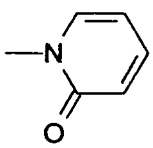 | H | H | H | H | 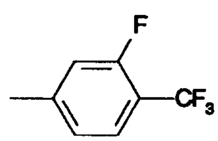 | O |

Tabelle 1: (Fortsetzung)

| Het | $R^1$ | $R^2$ | $R^3$ | $R^4$ | $R^5$ | $R^6$ | X |
|---|---|---|---|---|---|---|---|
| 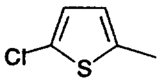 | 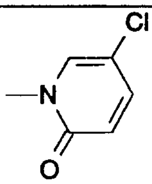 | H | H | H | H | 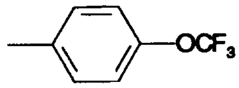 | O |
| 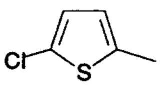 | 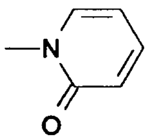 | H | H | H | H | 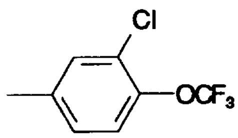 | O |
| 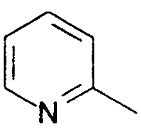 | 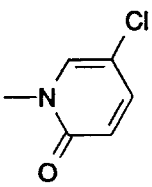 | H | H | H | H | 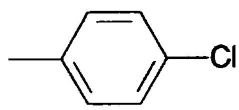 | O |
| 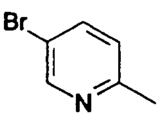 | 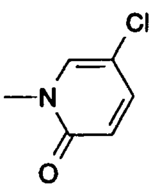 | H | H | H | H | 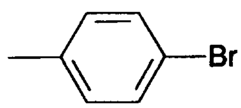 | O |
| 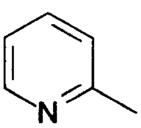 | 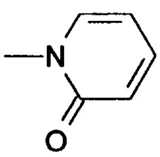 | H | H | H | H | 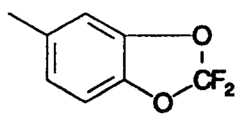 | O |
| 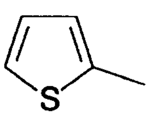 | 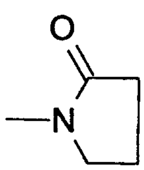 | H | H | H | H | 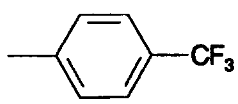 | O |

Tabelle 1: (Fortsetzung)

| Het | $R^1$ | $R^2$ | $R^3$ | $R^4$ | $R^5$ | $R^6$ | X |
|---|---|---|---|---|---|---|---|
| 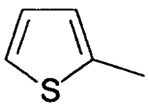 | 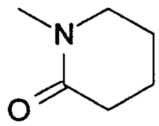 | H | H | H | H | 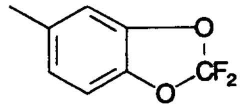 | O |
| 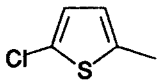 | 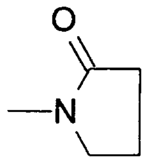 | H | H | H | H | 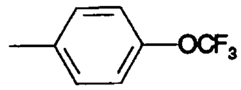 | O |
| 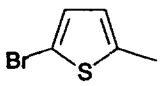 | 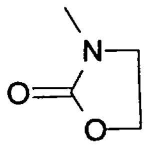 | H | H | H | H | 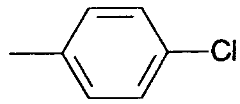 | O |
| 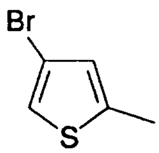 | 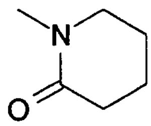 | H | H | H | H | 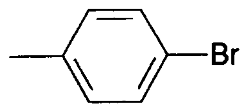 | O |
| 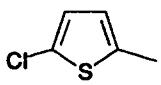 | 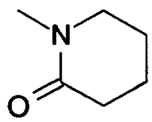 | H | H | H | H | 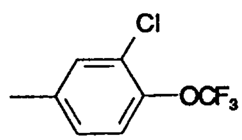 | O |
| 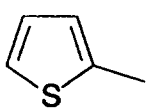 | 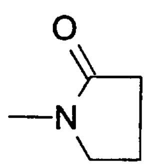 | H | H | H | H | 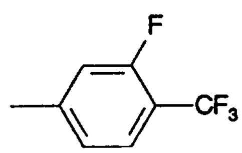 | O |

Tabelle 1: (Fortsetzung)

| Het | $R^1$ | $R^2$ | $R^3$ | $R^4$ | $R^5$ | $R^6$ | X |
|---|---|---|---|---|---|---|---|
| 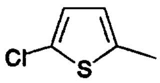 | 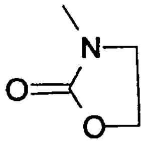 | H | H | H | H | 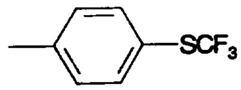 | O |
| 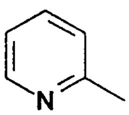 | 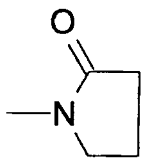 | H | H | H | H | 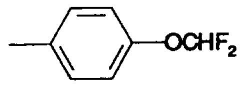 | O |
| 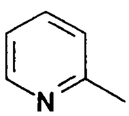 | 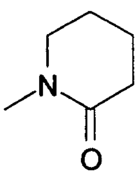 | H | H | H | H | 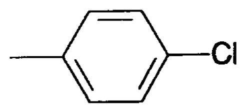 | O |
| 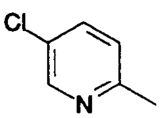 | 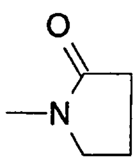 | H | H | H | H | 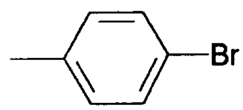 | O |
| 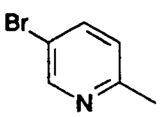 | 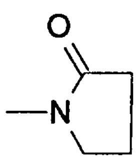 | H | H | H | H | 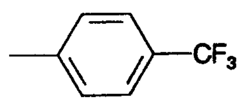 | O |
| 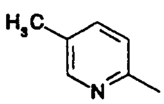 | 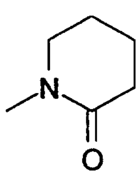 | H | H | H | H | 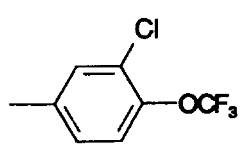 | O |

Tabelle 1 (Fortsetzung)

| Het | $R^1$ | $R^2$ | $R^3$ | $R^4$ | $R^5$ | $R^6$ | X |
|---|---|---|---|---|---|---|---|
| 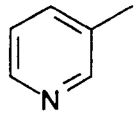 | 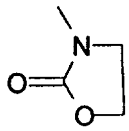 | H | H | H | H | 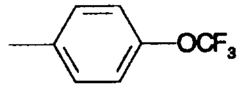 | O |
| 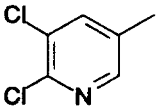 | 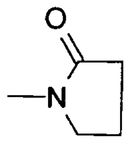 | H | H | H | H | 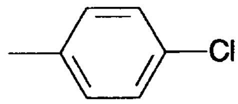 | O |
| 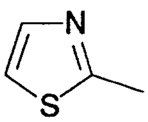 | 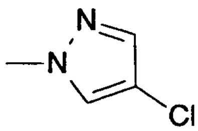 | H | H | H | H | 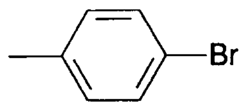 | O |
| 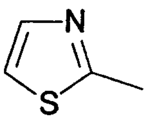 | 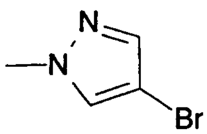 | H | H | H | H | 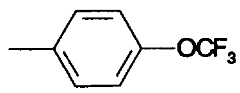 | O |
| 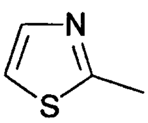 | 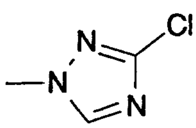 | H | H | H | H | 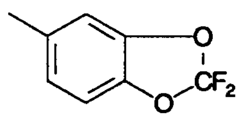 | O |
| 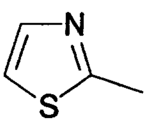 | 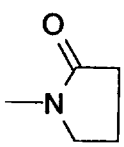 | H | H | H | H | 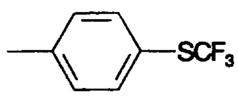 | O |
| 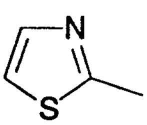 | 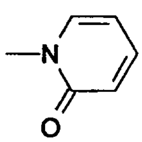 | H | H | H | H | 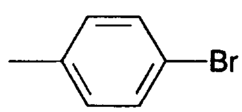 | O |

Tabelle 1 (Fortsetzung)

| Het | $R^1$ | $R^2$ | $R^3$ | $R^4$ | $R^5$ | $R^6$ | X |
|---|---|---|---|---|---|---|---|
| 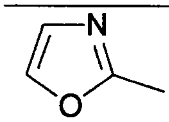 | 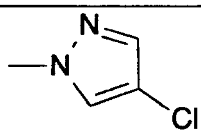 | H | H | H | H | 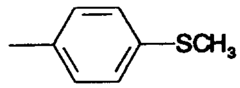 | O |
| 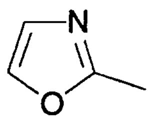 | 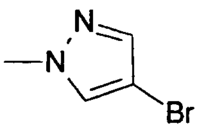 | H | H | H | H | 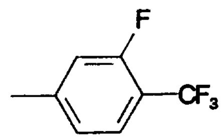 | O |
| 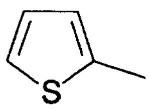 | 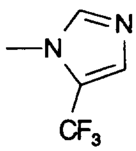 | H | H | H | H | 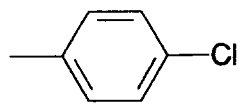 | O |
| 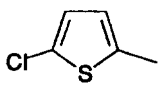 | 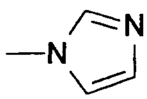 | H | H | H | H | 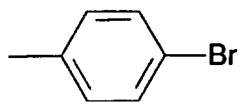 | O |
| 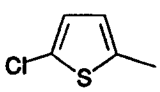 | 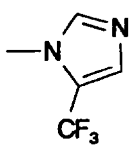 | H | H | H | H | 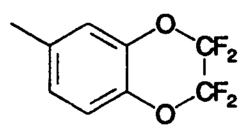 | O |
| 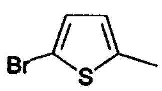 | 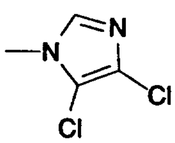 | H | H | H | H | 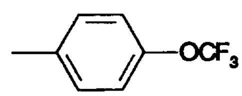 | O |
| 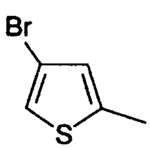 | 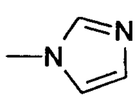 | H | H | H | H | 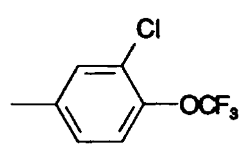 | O |

Tabelle 1 (Fortsetzung)

| Het | $R^1$ | $R^2$ | $R^3$ | $R^4$ | $R^5$ | $R^6$ | X |
|---|---|---|---|---|---|---|---|
| 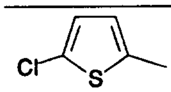 | 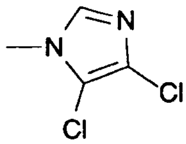 | H | H | H | H | 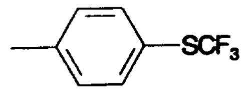 | O |
| 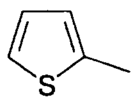 | 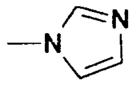 | H | H | H | H | 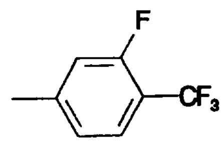 | O |
| 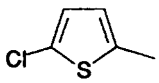 | 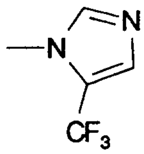 | H | H | H | H | 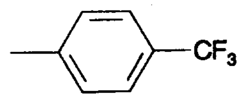 | O |
| 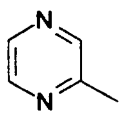 | 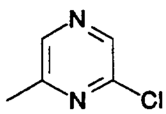 | H | H | H | H | 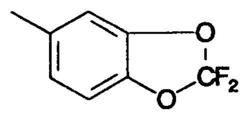 | O |
| 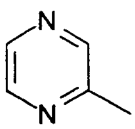 | 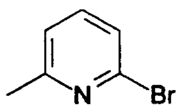 | H | H | H | H | 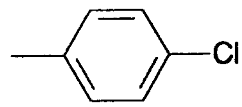 | O |
| 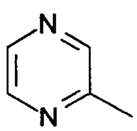 | 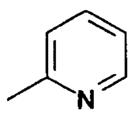 | H | H | H | H | 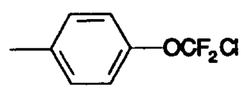 | O |
| 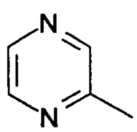 | 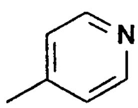 | H | H | H | H | 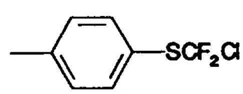 | O |

Tabelle 1 (Fortsetzung)

| Het | $R^1$ | $R^2$ | $R^3$ | $R^4$ | $R^5$ | $R^6$ | X |
|---|---|---|---|---|---|---|---|
| 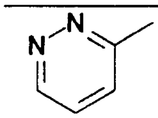 | 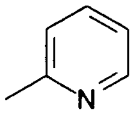 | H | H | H | H | 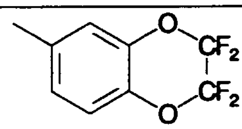 | O |
| 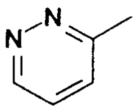 | 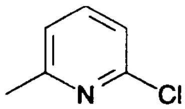 | H | H | H | H | 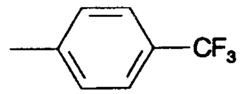 | O |
| 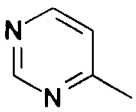 | 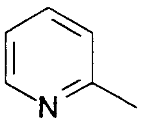 | H | H | H | H | 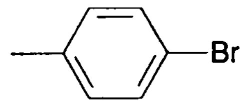 | O |
| 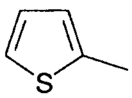 | 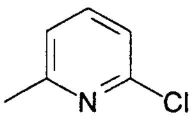 | H | H | H | H | 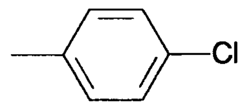 | O |
| 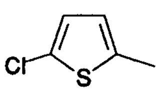 | 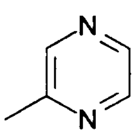 | H | H | H | H | 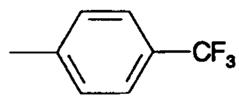 | O |
| 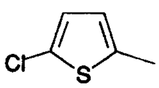 | 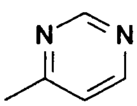 | H | H | H | H | 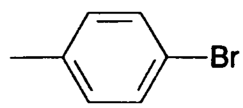 | O |
| 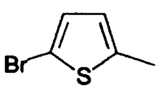 | 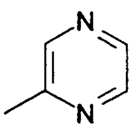 | H | H | H | H | 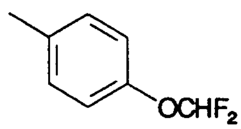 | O |

Tabelle 1 (Fortsetzung)

| Het | $R^1$ | $R^2$ | $R^3$ | $R^4$ | $R^5$ | $R^6$ | X |
|---|---|---|---|---|---|---|---|
| 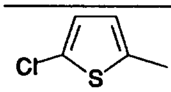 | 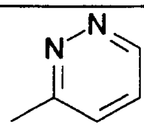 | H | H | H | H | 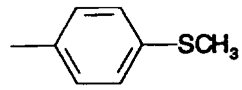 | O |
| 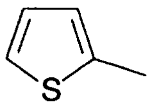 | 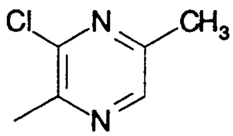 | H | H | H | H | 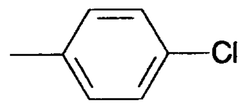 | O |
| 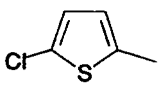 | 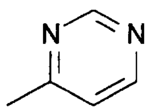 | H | H | H | $CH_3$ | 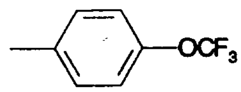 | O |
| 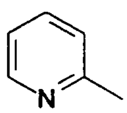 | 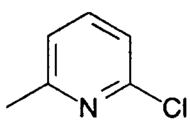 | H | H | H | H | 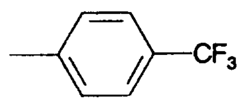 | O |
| 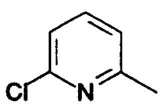 | 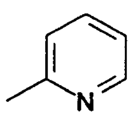 | H | H | H | H | 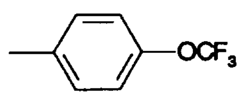 | O |
| 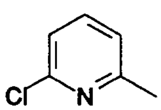 | 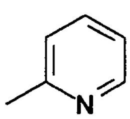 | H | H | H | H | 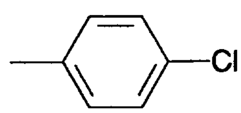 | O |
| 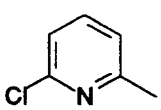 | 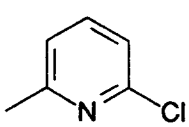 | H | H | $CH_3$ | H | 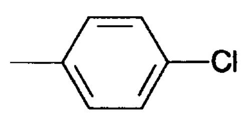 | O |

Tabelle 1 (Fortsetzung)

| Het | $R^1$ | $R^2$ | $R^3$ | $R^4$ | $R^5$ | $R^6$ | X |
|---|---|---|---|---|---|---|---|
| 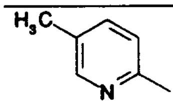 | 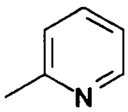 | H | H | H | H | 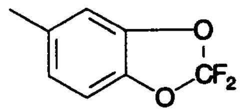 | O |
| 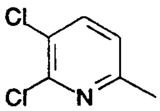 | 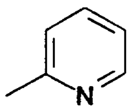 | H | H | H | H | 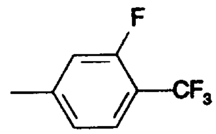 | O |
| 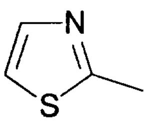 | 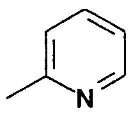 | H | H | H | H | 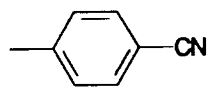 | O |
| 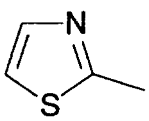 | 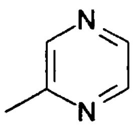 | H | H | H | H | 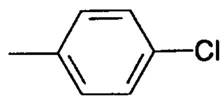 | O |
| 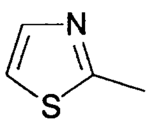 | 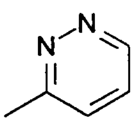 | H | H | H | H | 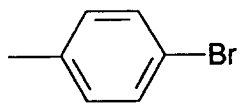 | O |
| 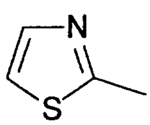 | 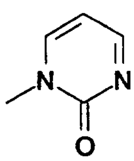 | H | H | H | H | 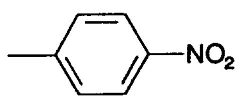 | O |
| 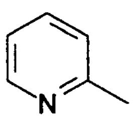 | 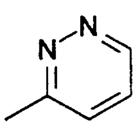 | H | H | H | H | 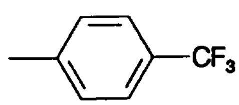 | O |

Tabelle 1: (Fortsetzung)

| Het | $R^1$ | $R^2$ | $R^3$ | $R^4$ | $R^5$ | $R^6$ | X |
|---|---|---|---|---|---|---|---|
| 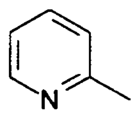 | 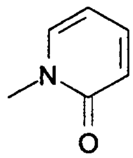 | H | H | H | H | 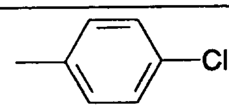 | O |
| 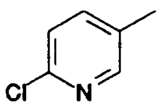 | 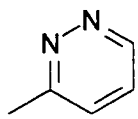 | H | H | H | H | 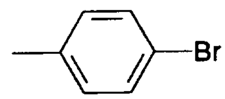 | O |
| 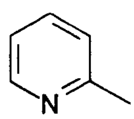 | 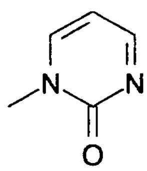 | H | H | H | H | 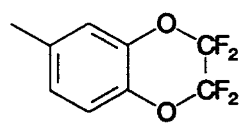 | O |
| 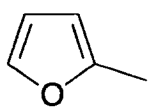 | 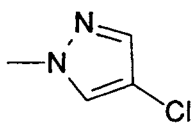 | H | H | H | H | 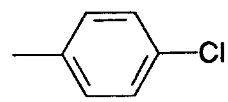 | O |
| 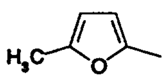 | 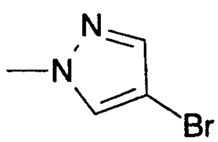 | H | H | H | H | 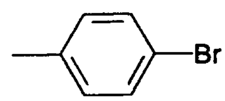 | O |
| 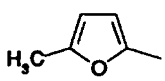 | 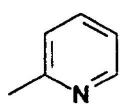 | H | H | H | H | 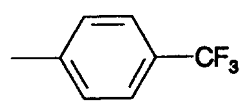 | O |
| 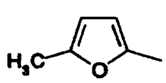 | 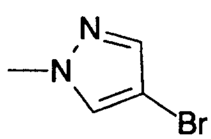 | H | H | H | H | 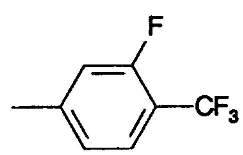 | O |

Tabelle 1: (Fortsetzung)

| Het | $R^1$ | $R^2$ | $R^3$ | $R^4$ | $R^5$ | $R^6$ | X |
|---|---|---|---|---|---|---|---|
| 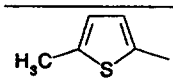 | 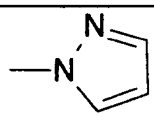 | H | H | H | H | 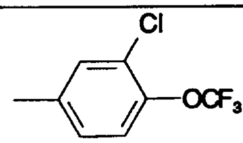 | O |
| 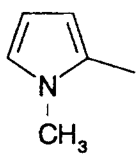 | 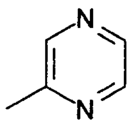 | H | H | H | H | 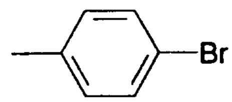 | O |
| 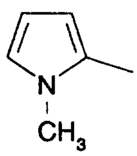 | 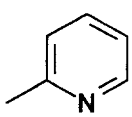 | H | H | H | H | 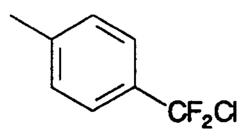 | O |
| 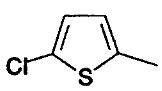 | 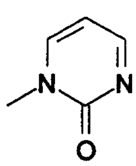 | H | H | H | H | 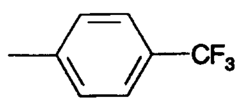 | O |
| 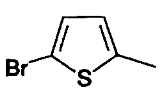 | 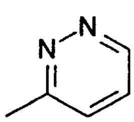 | H | H | H | H | 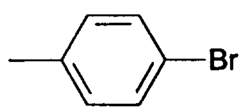 | O |
| 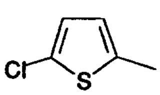 | 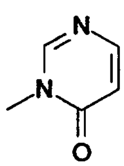 | H | H | H | H | 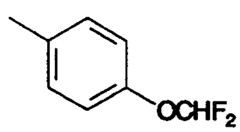 | O |

Tabelle 1: (Fortsetzung)

| Het | $R^1$ | $R^2$ | $R^3$ | $R^4$ | $R^5$ | $R^6$ | X |
|---|---|---|---|---|---|---|---|
| 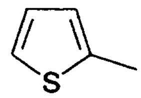 | 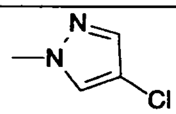 | H | H | H | H | 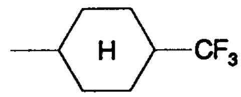 | O |
| 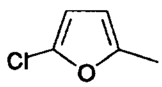 | 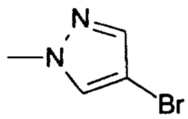 | H | H | H | H | 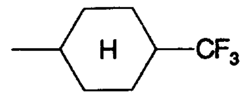 | O |
| 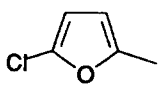 | 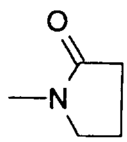 | H | H | H | H | 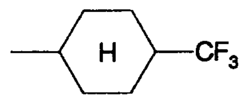 | O |
| 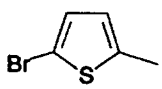 | 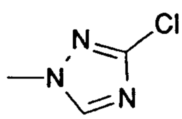 | H | H | H | H | 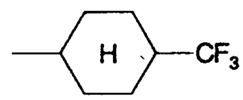 | O |
| 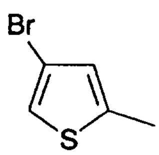 | 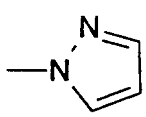 | H | H | H | H | 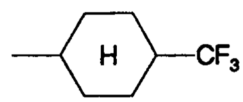 | S |
| 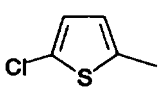 | 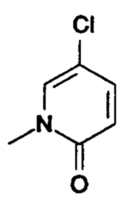 | H | H | H | H | 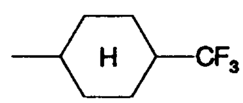 | O |
| 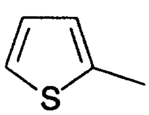 | 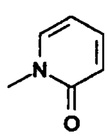 | H | H | H | H | 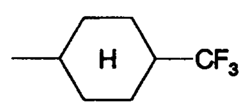 | O |

Tabelle 1 (Fortsetzung)

| Het | $R^1$ | $R^2$ | $R^3$ | $R^4$ | $R^5$ | $R^6$ | X |
|---|---|---|---|---|---|---|---|
| 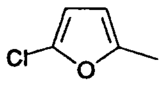 | 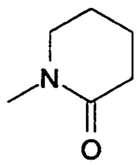 | H | H | H | H | 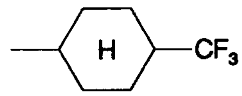 | O |
| 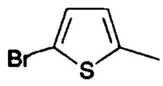 | 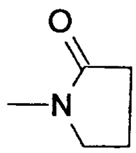 | H | H | H | H | 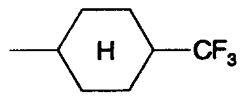 | O |
| 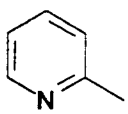 | 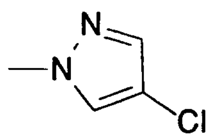 | H | H | H | H | 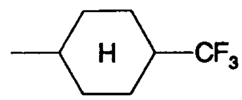 | O |
| 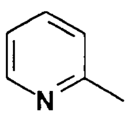 | 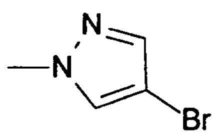 | H | H | H | H | 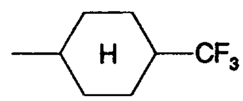 | O |
| 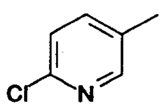 | 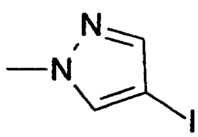 | H | H | H | H | 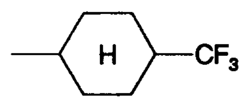 | O |
| 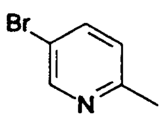 | 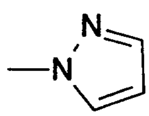 | H | H | H | H | 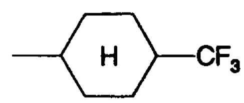 | O |
| 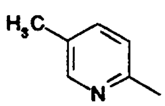 | 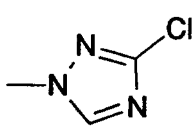 | H | H | H | H | 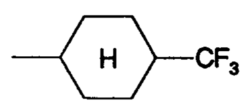 | O |

Tabelle 1: (Fortsetzung)

| Het | $R^1$ | $R^2$ | $R^3$ | $R^4$ | $R^5$ | $R^6$ | X |
|---|---|---|---|---|---|---|---|
| 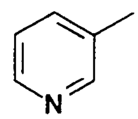 | 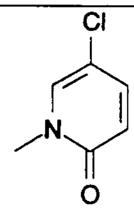 | H | H | H | H | 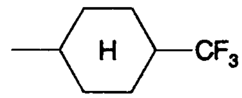 | O |
| 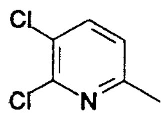 | 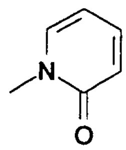 | H | H | H | H | 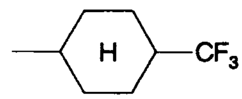 | O |
| 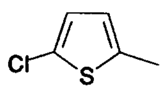 | 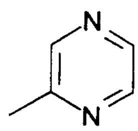 | H | H | H | H | 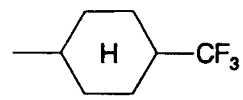 | O |
| 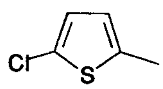 | 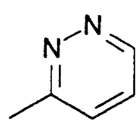 | H | H | H | H | 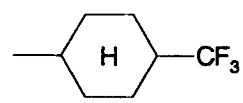 | O |
| 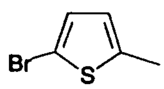 | 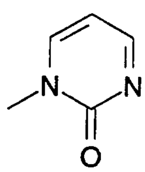 | H | H | H | H | 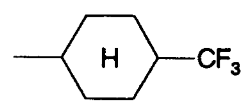 | O |
| 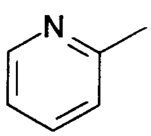 | 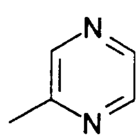 | H | H | H | H | 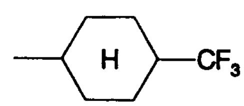 | O |

Tabelle 1: (Fortsetzung)

| Het | $R^1$ | $R^2$ | $R^3$ | $R^4$ | $R^5$ | $R^6$ | X |
|---|---|---|---|---|---|---|---|
| 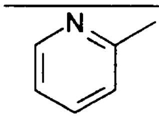 | 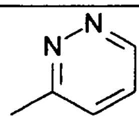 | H | H | H | H | 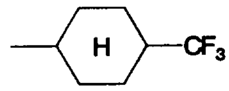 | O |
| 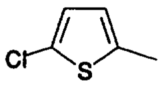 | 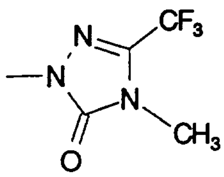 | H | H | H | H | 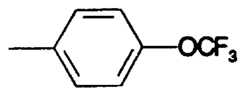 | O |
| 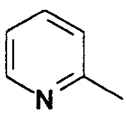 | 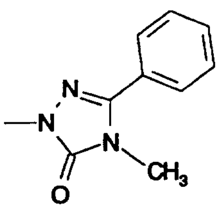 | H | H | H | H | 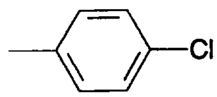 | O |
| 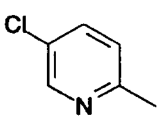 | 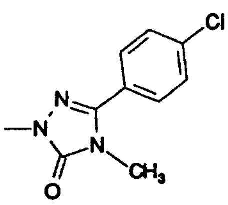 | H | H | H | H | 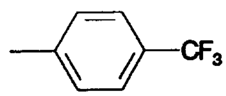 | O |
| 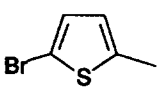 | 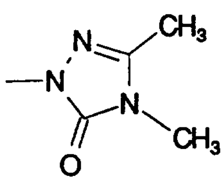 | H | H | H | H | 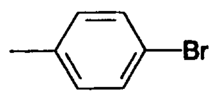 | O |
| 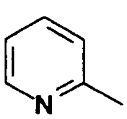 | 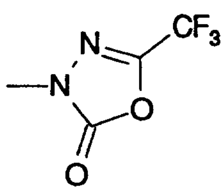 | H | H | H | H | 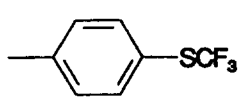 | O |

Verwendet man beispielsweise 3-Pyrid-4'-yl-pyrid-2''-yl-4,5-dihydropyrazol und 4-Trifluormethoxy-phenylisocyanat als Ausgangsstoffe, so läßt sich der Reaktionsablauf des erfindungsgemäßen Verfahrens (A) durch das folgende Formelschema darstellen:

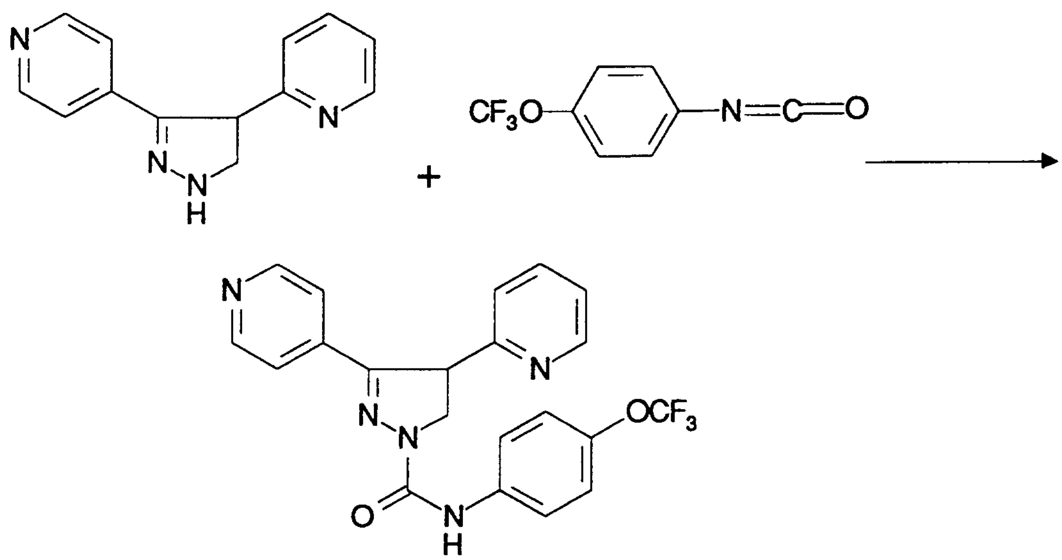

Verwendet man beispielsweise 3-Pyrid-4'-yl-4-pyrid-2''-yl-4,5-dihydropyrazol und N-(4-Trifluormethoxyphenyl)-carbamidsäurechlorid als Ausgangsstoffe, so läßt sich der Reaktionsablauf des erfindungsgemäßen Verfahrens (B) durch das folgende Formelschema darstellen:

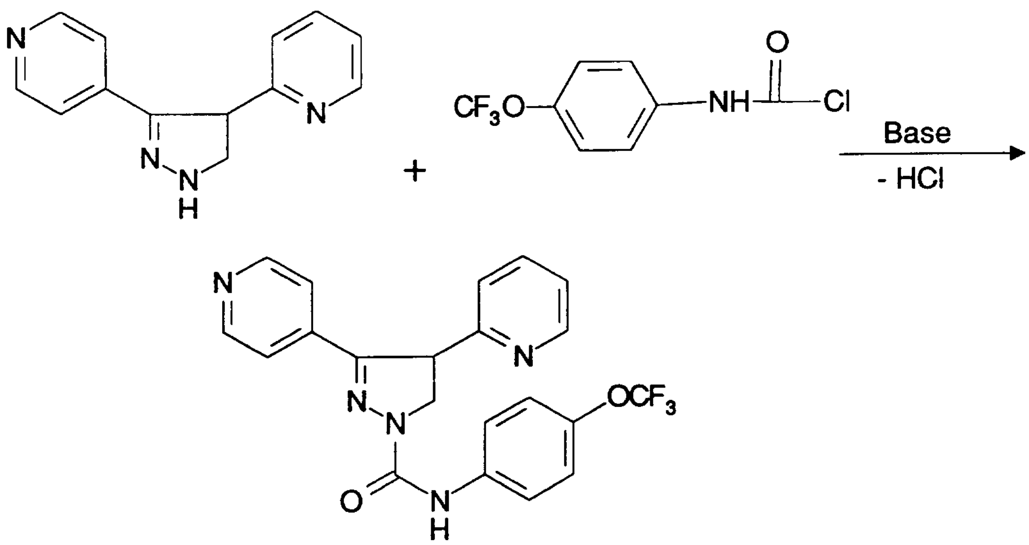


Die zur Durchführung der erfindungsgemäßen Verfahren (A) und (B) als Ausgangsstoffe benötigten Pyrazolinderivate sind durch die Formel (II) allgemein definiert. In dieser Formel (II) stehen $R^1$, $R^2$, $R^3$, $R^4$ und Het vorzugsweise für diejenigen Reste, die bereits im Zusammenhang mit der Beschreibung der erfindungsgemäßen Stoffe der Formel (I) als bevorzugt für diese Substituenten genannt wurden. Die Pyrazolinderivate der Formel (II) sind neu und können nach einem der folgenden Verfahren hergestellt werden: Sie werden durch Umsetzung von Verbindungen der Formel (VI)

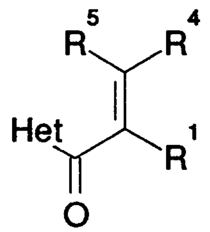

(VI)

in einem polaren organischen Lösungsmittel, vorzugsweise einem Alkanol bei Temperaturen von 20 bis 80 °C insbesondere bei 30 bis 60 °C mit Hydrazin-Hydrat hergestellt:

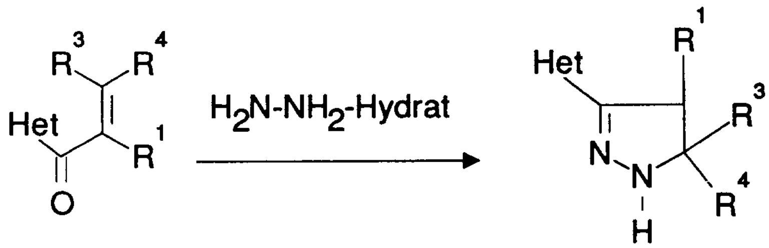


(II)

In Abhängigkeit von der Bedeutung der Substituenten $R^3$ und $R^4$ ergeben sich dabei folgende Herstellungsvarianten der Ausgangsverbindungen der Formel (VI)

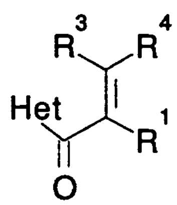

(VI)

(a)
$R^3$ und $R^4$ in der Formel (VI) stehen für Wasserstoff

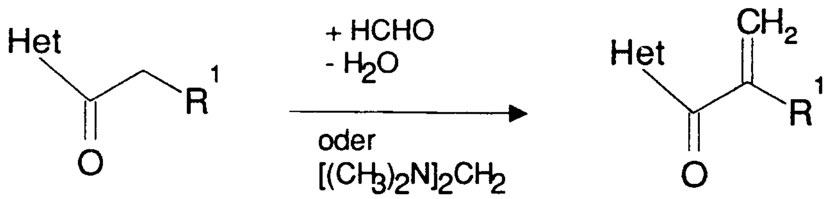


(VIa) (VIb)

Hierbei werden Verbindungen der Formel (VIa) entweder in einem polaren organischen Lösungsmittel, vorzugsweise einem Alkanol und insbesondere in Ethanol oder Methanol mit einer Formalinlösung unter Zusatz geringer - Mengen einer organischen Base, insbesondere von Piperidin sowie Zusatz von Eisessig oder mit Bis-(dimethylamino)-methan in Essigsäureanhydrid umgesetzt.
(b) In der Formel (VI) steht $R^3$ für Alkyl oder Aryl und $R^4$ steht für Wasserstoff

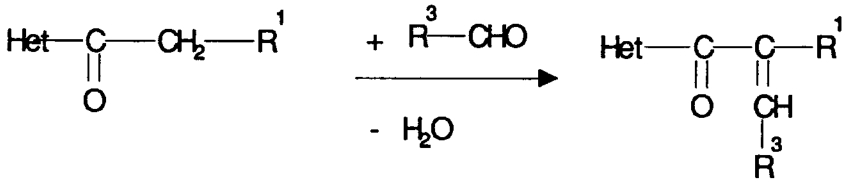


(VIa) (VIc)

Die Verfahrensbedingungen entsprechen denjenigen der Umsetzung mit Formaldehyd.
(c) In der Formel (VI) stehen $R^3$ und $R^4$ für Alkyl:

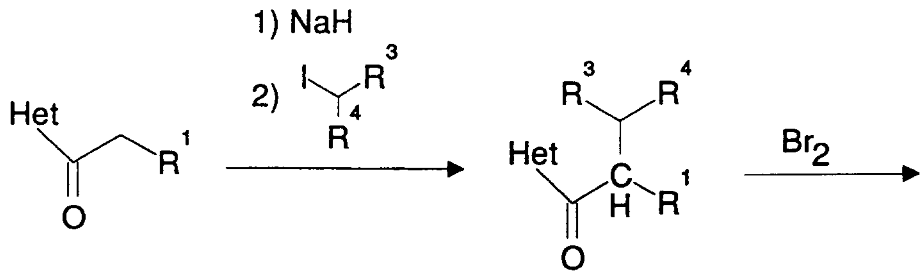


(VIa) (VII)

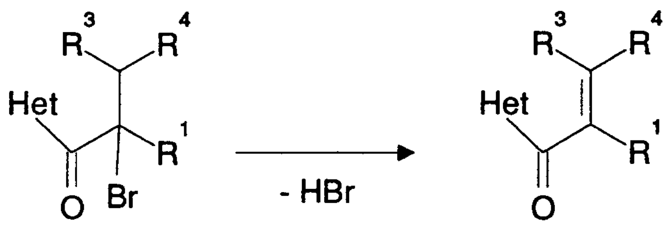


(VIII) (IX)

Hierbei wird die Verbindung (VI) zunächst mit einer starken Base in das Salz übergeführt und anschließend mit einem Halogenid, insbesondere einem Iodid der Formel

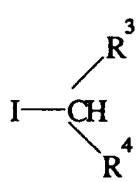

umgesetzt.

Die dabei entstehende Verbindung der Formel (VII) wird bromiert und anschließend wird durch Zusatz einer Base unter HBr-Eliminierung das Zwischenprodukt der Formel (IX) hergestellt.

Verbindungen der Formel (IIa), in welcher $R^3$ und $R^4$ für Wasserstoff stehen, werden außerdem durch Umsetzung von Verbindungen der Formel (VId)

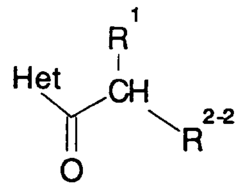

(VId)

in welcher

Het und $R^1$ die oben angegebene Bedeutung haben und

$R^{2-2}$ für Wasserstoff oder Alkyl steht,

erhalten, indem man diese zunächst in einer 1. Stufe in einem polaren organischen Lösungsmittel, vorzugsweise Acetonitril, bei Temperaturen von 10 bis 100 °C, insbesondere bei 20 bis 80 °C mit einem Mol N,N-Dimethylmethylenimmoniumchlorid der Formel (XI)

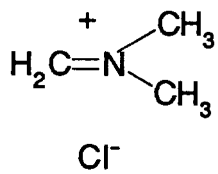

(XI)

gegebenenfalls am Rückfluß erhitzt, wobei das intermediär auftretende Zwischenprodukt der Formel (XII)

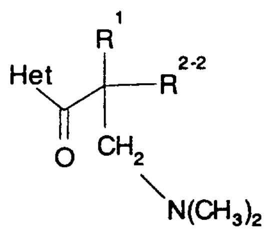

(XII)

gegebenenfalls isoliert und anschließend in einer zweiten Stufe in einem polaren organischen Lösungsmittel, vorzugsweise einem Alkohol bei Temperaturen von 20 bis 80 °C insbesondere bei 30 bis 60 °C mit Hydrazin-Hydrat zu Verbindungen der Formel (II) cyclisiert.

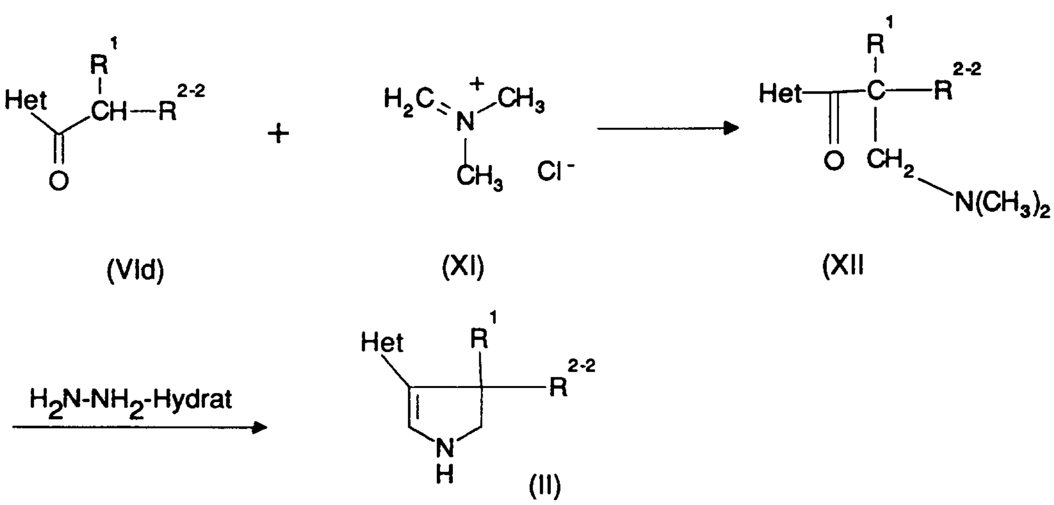


(VId) (XI) (XII

(II)

Verbindungen der Formel (XII) können außerdem hergestellt werden (a) durch Umsetzung von Verbindungen der Formel (VId) in einem Alkanol bei 30 bis 80 °C mit Dimethylaminhydrochlorid und Paraformaldehyd

und anschließendes Fällen des Salzes mit einem unpolaren Lösungsmittel, z.B. Ether oder (b) durch Umsetzung von Verbindungen der Formel (VId) in Chloroform, Toluol oder Acetonitril bei 30 bis 80 °C mit Bis(dimethylamino)-methan:

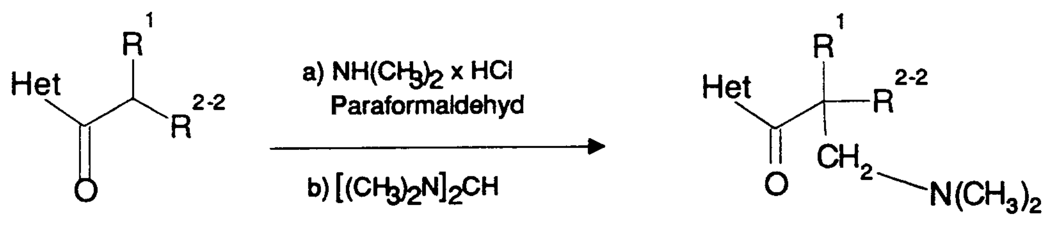


(VId) (XII)

Die Verbindungen der Formel (VIa) und (VIb) sind teilweise neu. Sie können nach einem der im folgenden aufgeführten Verfahren erhalten werden:

(a) Verbindungen der Formel (VIa) und (VIb), bei welchen $R^1$ für einen über Stickstoff gebundenen Hetrocyclus steht, werden erhalten, wenn man Verbindungen der Formel (X)

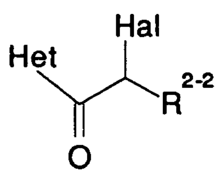

(X)

worin

$R^{2-2}$ für Wasserstoff oder Alkysl steht,

Het die oben angegebene Bedeutung hat und

Hal für Halogen, insbesondere Brom steht mit Heterocyclen der Formel (XIII)

$R^{1-1}$-H (XIII)

worin

$R^{1-1}$ für einen gegebenenfalls substituierten Pyrrolyl-, Pyrazolyl-, Imidazolyl-, Pyridonyl-, Pyrimidonyl-, 1,2,3-Triazolyl-, 1,2,4-Triazolyl- oder Tetrazolyl-Rest steht

oder für einen unsubstituierten oder einfach oder zweifach, gleich oder verschieden substituierten und über Stickstoff gebundenen Azolinon, Azolinthion- oder Azoliniminrest aus der Reihe

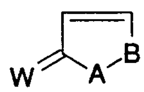 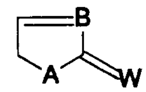 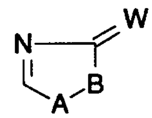 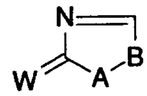

($R^1$-b) ($R^1$-c) ($R^1$-e) ($R^1$-f)

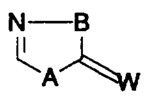 oder 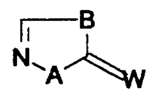

($R^1$-h) ($R^1$-i)

worin

eine der Gruppen A oder B für Stickstoff steht und jeweils die andere (A oder B) für Sauerstoff, Schwefel oder für die Gruppe - NAlkyl($C_1$-$C_4$-) oder für eine Methylengruppierung -$CH_2$- oder für eine - CH-Gruppe steht und

W für Sauerstoff oder Schwefel steht,

oder für einen unsubstituierten oder einfach bis dreifach substituierten Heterocyclus aus der Reihe

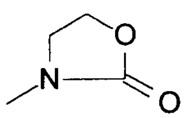, 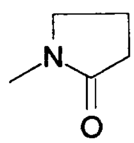, 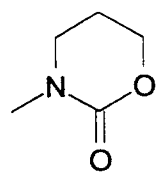, 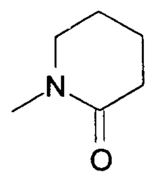,

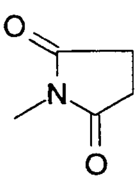, 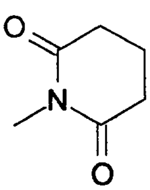

steht

unter Zuhilfenahme einer organischen oder anorganischen Base unter Halogenwasserstoffeleminierung umsetzt.

(b) Verbindungen der Formel (VIa) und (VIb), bei welchen $R^1$ für einen über Kohlenstoff gebundenen Heterocyclus steht, werden erhalten

(b-$\alpha$) wenn man Verbindungen der Formel (XIV)

Het-$R^{12}$ (XIV)

$R^{12}$ für eine Cyano- oder Alkoxycarbonylgruppe, vorzugsweise für Cyano, Methoxycarbonyl oder Ethoxycarbonyl steht,

mit Heterocyclen dere Formel (XV)

$R^{1-2}$-$CH_3$ (XV)

in welcher

$R^{1-2}$ für einen gegebenenfalls substituierten Pyridinyl-, Pyrimidinyl-, Pyrazinyl-, Pyridazinyl- oder Triazinyl-Rest steht

entweder in Gegenwart von Alkyl-Lithiumverbindungen, wie beispielsweise n-Butyl-Lithium in

einem inerten organischen Lösungsmittel, wie beispielsweise Tetrahydrofuran bei -50 °C bis +10 °C, insbesondere - 30 °C bis -15 °C , gegebenenfalls in Anwesenheit einer Schutzgasatmosphäre, wie z.B. Argon, umsetzt oder mit Metallhydriden, wie beispielsweise Natriumhydrid in Dimethylformamid oder mit starken Basen, wie beispielsweise Kaliumhydroxid umsetzt oder

(b-$\beta$) wenn man Heterocyclen der Formel (XVI)

Het-H (XVI)

in welcher

Het die oben angegebene Bedeutung hat mit Verbindungen der Formel (XVII)

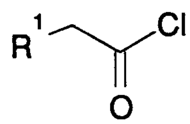

**(XVII)**

in welcher

$R^1$ die oben angegebene Bedeutung hat,

gegebenenfalls in Gegenwart eines inerten organischen Lösungsmittels, wie beispielsweise Hexan oder Chloroform mit Lewis-Säuren, wie beispielsweise Aluminiumchlorid bei Temperaturen zwischen 30 °C und 130 °C, umsetzt.

Die Verbindungen der Formeln (X), (XIII), (XIV), (XV), (XVI) und (XVII) sind allgemein bekannte Verbindungen der organischen Chemie.

Die zur Durchführung des erfindungsgemäßen Verfahrens (B) benötigten Verbindungen der Formel (IV) sind ebenfalls allgemein bekannte Verbindungen der organischen Chemie.

Die Wirkstoffe eignen sich zur Bekämpfung von tierischen Schädlingen, vorzugsweise Arthropoden und Nematoden, insbesondere Insekten und Spinnentieren, die in der Landwirtschaft, in der Tierhaltung, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa sppl. Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculamx thurberiella, Phyllocnistis citrella, Agrotis spp., Euxoa spp., Feltia spp., Earias insulana, Heliothis spp., Spodoptera exigua, Mamestra brassicae, Panolis flammea,

Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Hydrotaea spp., Haematobia spp., Glossina spp., Melophagus spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp., Ctenocephalides spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Ornithonyssus spp., Eriophyes ribis, Phyllocoptruta oleivora, Boophilus spp., Rhipicephalus spp., Amblyomma spp., Hyalomma spp., Ixodes spp., Dermacentor spp., Haemaphysalis spp., Otobius spp., Psoroptes spp., Chorioptes spp., Sarcoptes spp., Psorergates spp., Demodex spp., Notoedres spp., Tarsonemus spp., Bryobia praetiosa, Panonychus spp., Tetranychus spp..

Die erfindungsgemäßen Wirkstoffe wirken nicht nur gegen Pflanzen-, Hygiene-und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Tabaknospenraupe (Heliothis virescens) oder gegen die Raupen der Kohlschabe (Plutella maculipennis) einsetzen.

Darüberhinaus lassen sie sich auch mit besonders gutem Erfolg zur Bekämpfung von parasitisch lebenden Warmblüterschädlingen, wie beispielsweise gegen die Larven der Goldfliege (Lucilia cuprina) oder gegen Musca domestica und gegen Periplaneta americana einsetzen.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste

Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke), sowie eine Anwendung in Form der sogenannten Umgebungsbehandlung möglich.

Die nachfolgenden Beispiele beschreiben die Herstellung und die Verwendung erfindungsgemäßer Wirkstoffe ohne sie darauf zu beschränken.

Herstellungsbeispiele:

Beispiel 1

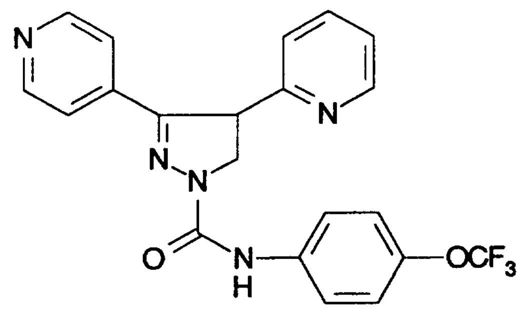

3,9 g (0,0174 Mol) 3-Pyrid-4'-yl-4-pyrid-2''-yl-4,5-dihydropyrazol werden bei 50 °C in 20 ml wasserfreiem Acetonitril gelöst, 3,25 g (0,016 Mol) 4-Trifluormethoxyphenylisocyanat zugegeben und mit 2 Tropfen Triethylamin versetzt. Man läßt die Mischung bei 20 °C 2 Stunden stehen und engt dann im Vakuum ein. Der Rückstand wird mit 20 ml Diethylether versetzt und nach 2 Stunden wird der kristalline Niederschlag abgesaugt.

Man erhält 1,1 g 3-Pyrid-4'-yl-4-pyrid-2''-yl-4,5-dihydropyrazol-1-carbonsäure-4'''-trifluormethoxyanilid als farblose Kristalle mit dem Schmelzpunkt 172 °C.

Analog zu Beispiel 1 und gemäß den allgemeinen Angaben zur Herstellung werden die nachfolgend in Tabelle 2 aufgeführten Endprodukte der Formel (I) erhalten:

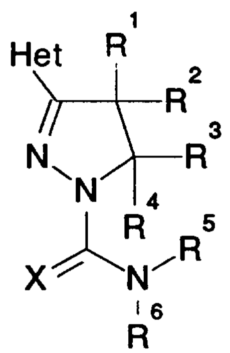

(I)

Tabelle 2:

| Bsp. Nr. | Het | $R^1$ | $R^2$ | $R^3$ | $R^4$ | $R^5$ | $R^6$ | X | physik. Konst. [Fp:°C] |
|---|---|---|---|---|---|---|---|---|---|
| 2 | 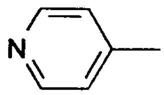 | 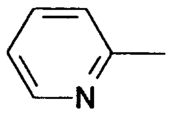 | H | H | H | H | 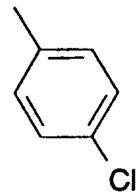 | O | 159 |
| 3 | 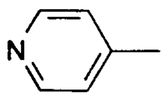 | 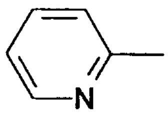 | H | H | H | H | 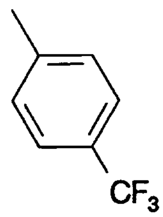 | O | 206 |
| 4 | 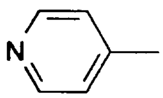 | 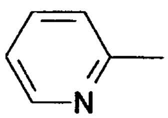 | H | H | H | H | 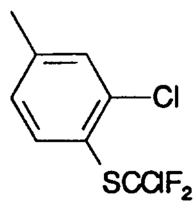 | O | 208 |
| 5 | 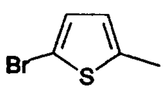 | 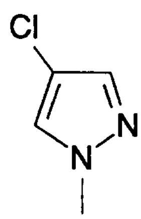 | H | H | H | H | 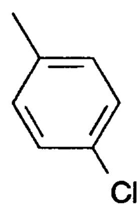 | O | 193 |

Tabelle 2:

| Bsp. Nr. | Het | $R^1$ | $R^2$ | $R^3$ | $R^4$ | $R^5$ | $R^6$ | X | physik. Konst. [Fp:°C] |
|---|---|---|---|---|---|---|---|---|---|
| 6 | 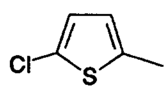 | 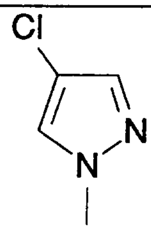 | H | H | H | H | 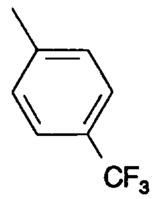 | O | 171 |
| 7 | 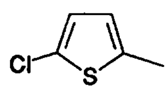 | 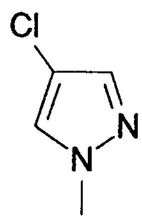 | H | H | H | H | 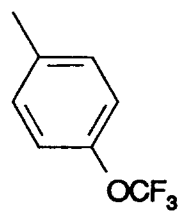 | O | 179 |
| 8 | 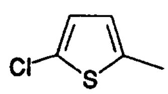 | 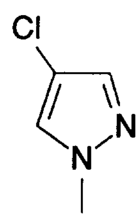 | H | H | H | H | 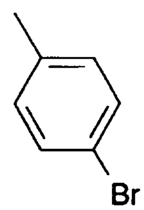 | O | 195 |
| 9 | 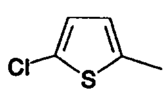 | 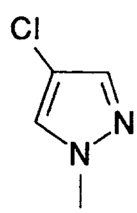 | H | H | H | H | 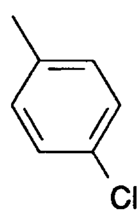 | O | >230 |

Tabelle 2:

| Bsp. Nr. | Het | $R^1$ | $R^2$ | $R^3$ | $R^4$ | $R^5$ | $R^6$ | X | physik. Konst. [Fp:°C] |
|---|---|---|---|---|---|---|---|---|---|
| 10 | 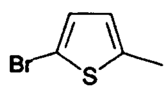 | 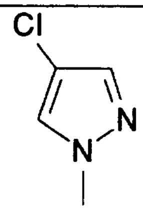 | H | H | H | H | 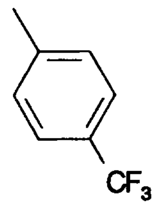 | O | 177 |
| 11 | 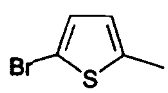 | 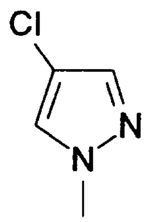 | H | H | H | H | 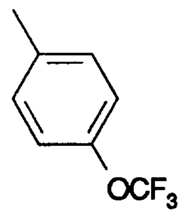 | O | 186 |
| 12 | 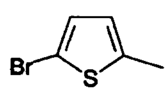 | 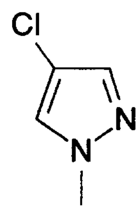 | H | H | H | H | 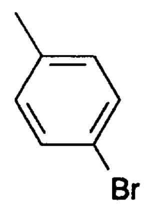 | O | 187 |

Herstellung der Vorprodukte:

Beispiel (II-1)

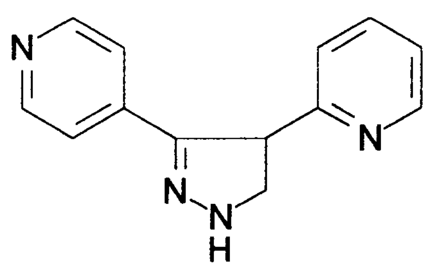

23,8 g (0,12 Mol) α-Pyrid-2-yl-4-acetyl-pyridin werden in 80 ml Acetonitril bei 20 bis 30 °C gelöst, mit 15 g (0,15 Mol) N,N-Dimethylmethylenimmoniumchlorid versetzt und 1 1/2 Stunden auf 80 °C erhitzt. Die Vollständigkeit der Umsetzung wird dünnschichtchromatographisch kontrolliert. Man kühlt auf 50 °C ab und gibt dann 20 ml Hydrazinhydrat zu. Es wird noch 30 Minuten bei 20 - 40 °C nachgerührt, anschließend im Vakuum auf ca. ein Drittel eingeengt, mit 100 ml Wasser versetzt und mit 150 ml Diethylether extrahiert.

Die Etherphase wird abgetrennt, getrocknet ($MgSO_4$) und im Vakuum eingeengt.

Man erhält 18,3 g 3-Pyrid-4'-yl-4-pyrid-2''-yl-4,5-dihydropyrazol als braunes Öl, das ohne weitere Reinigung weiter eingesetzt wird.

[$^{1}$H-NMR*($CDCl_3$); $\delta$ = 4,70-4,755 (1H, m), 4,06-4,16 (1H, m); 3,77-3,82 (1H, m)].

Analog Beispiel (II-1) und gemäß den allgemeinen Angaben zur Herstellung werden die nachstehend in Tabelle 3 aufgeführten Vorprodukte der Formel (II) erhalten:

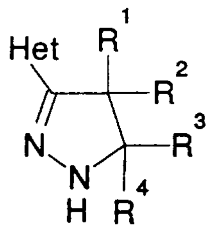

(II)

Tabelle 3:

| Bsp.-Nr. | Het | $R^1$ | $R^2$ | $R^3$ | $R^4$ | physik. Konst. [Fp.:°C] |
|---|---|---|---|---|---|---|
| II-2 | 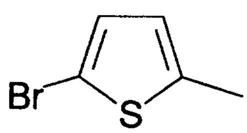 | 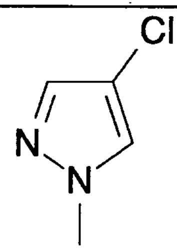 | H | H | H | 139 |

*H-NMR Spektren wurden in deuteriertem Chloroform ($CDCl_3$) mit Tetramethylsilan (TMS) als innerem Standard aufgenommen. Angegeben ist die chemische Verschiebung $\delta$ in ppm.

Herstellung der Ausgangsprodukte:

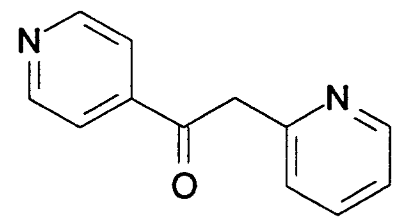

Unter Schutzgasatmosphäre (Argon) werden 18,6 g (0,2 Mol) α-Picolin bei Raumtemperatur in 200 ml wasserfreiem Tetrahydrofuran gelöst. Bei -70 °C werden dann 80 ml n-Butyllithium-Lösung in Hexan (Gehalt: 2,5 mol/l) zugetropft. Die Reaktionsmischung wird anschließend bei 0 °C 1 Stunde gerührt und dann, ebenfalls bei 0 °C, 20,8 g (0,2 Mol) 4-Cyanopyridin gelöst in 50 ml wasserfreiem Tetrahydrofuran zugetropft. Die Temperatur steigt dabei auf 30 °C, anschließend wird noch 30 Minuten bei 20 °C nachgerührt. Die Reaktionsmischung wird dann mit 100 ml Wasswer versetzt, dann mit 200 ml Diethylether extrahiert, die organische Phase abgetrennt, getrocknet ($MgSO_4$) und das Lösungsmittel im Vakuum abgezogen. Der Rückstand wird dann mit einer Mischung aus Petrolether-Diethylether1:1 verrührt und die entstandenen Kristalle abgesaugt.

Man erhält 28,76 g (72,5 % der Theorire) 2-(4-Methyl-2-pyridinyl)-1-(4-pyridinyl)ethanon als gelbe Kristalle mit dem Schmelzpunkt 112 °C.

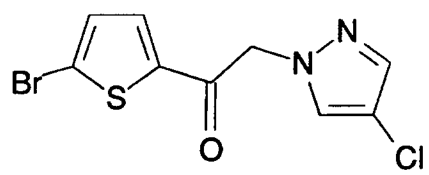

11,63 g (0,118 Mol) 4-Chlorpyrazol-hydrochlorid werden in 100 ml absolutem Acetonitril suspendiert und mit 34,5 g (0,25 Mol) gepulvertem Kaliumcarbonat versetzt. Anschließend tropft man 32,37 g (0,118 Mol) 2-Brom-5-(2-bromacetyl)thiophen gelöst in 50 ml absolutem Acetonitril zu und erwärmt 1 Stunde unter Rückfluß. Das Reaktionsgemisdch wird in 300 ml Wasser eingerührt und mit Methylenchlorid extrahiert. Es wird über Natriumsulfat getrocknet, das Lösungsmittel im Vakuum abgedampft und der Rückstand aus Essigester umkristallisiert.

Man erhält 4,9 g (ca. 16 % der Theorie) vom 2-(4-Chlor-1-pyrazolyl)-1-(2-brom-5-thienyl)-ethanon vom Schmelzpunkt 127 °C.

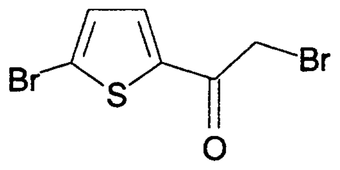

Zu einer Lösung aus 24,8 g (0,121 Mol) 2-Acetyl-5-brom-thiophen in 120 ml Dichlormethan werden bei 0 bis 5 °C 19,36 g (0,121 Mol) Brom zugetropft und der Ansatz 1 Stunde bei dieser Temperatur gerührt. Nach dem Erwärmen auf Raumtemperatur wird das Reaktionsgemisch in 200 ml Eiswasser eingerührt und mit Dichlormethan extrahiert. Die organische Phase wird zweimal mit Eiswasser gewaschen, über Natriumsulfat getrocknet und das Lösungsmittel im Vakuum abgezogen.

Man erhält 32,4 g (94 % der Theorie) 2-Brom-5-(2-bromacetyl)-thiophen vom Schmelzpunkt 79 °C.

Anwendungsbeispiele

In den folgenden Anwendungsbeispielen wurden die nachstehend aufgeführten Verbindungen als Vergleichssubstanzen eingesetzt.

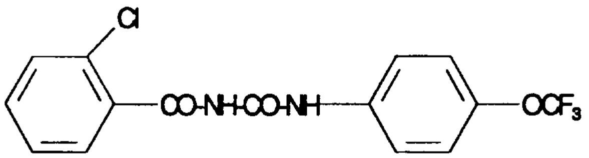

(A)

Triflumuron = 2-Chlor-N[[[4-(trifluormethoxy)-phenyl]-amino]-carbonyl]-benzamid
(bekannt aus: DE-A 2601780)

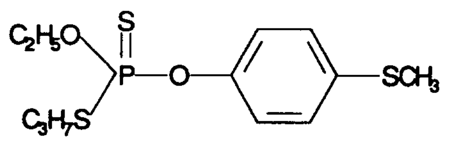

(B)

Sulprofos = O-(Ethyl-O-(4-methylthio)-phenyl)-S-propyldithiophosphat
(bekannt aus: DE-A 2111414)

Beispiel A

Phaedon-Larven-Test

| | | |
|---|---|---|
| Lösungsmittel:<br>Emulgator: | 7 Gewichtsteile<br>1 Gewichtsteil | Dimethylformamid<br>Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 6, 9, 10, 11 und 12.

Beispiel B

Plutella -Test

| | | |
|---|---|---|
| Lösungsmittel:<br>Emulgator: | 7 Gewichtsteile<br>1 Gewichtsteil | Dimethylformamid<br>Alkylarylpolyglykolether |

Zur Herstellung einer zweckmäßigen Wirkstoffzubereitung vermischt man 1 Gewichtsteil Wirkstoff mit der angegebenen Menge Lösungsmittel und dere angegebenen Menge Emulgator und verdünnt das Konzentrat mit Wasser auf die gewünschte Konzentration.

Kohlblätter (Brassica oleracea) werden durch tauchen in die Wirkstoffzubereitung der gewünschten Konzentration behandelt und mit Raupen der Kohlschabe (Plutella maculipennis) besetzt, solange die Blätter feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem test zeigen z.B. die folgenden Verbindungden der Herstellungsbeispiele überlegene Wirksamkeit gegenüber dem Stand der Technik: 7.

Beispiel C

Heliothis virescens-Test

| | | |
|---|---|---|
| Lösungsmittel:<br>Emulgator: | 7 Gewichtsteile<br>1 Gewichtsteil | Dimethylformamid<br>Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit der Tabakknospenraupe (Heliothis virescens) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 5, 6, 7, 8, 9, 10, 11 und 12.

Beispiel D

Blowfly-Larven-Test

Testtiere: Lucilia cuprina-Larven
Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Emulsionskonzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird die Wirksamkeit der Wirkstoffzubereitung ermittelt. Dabei bedeutet 100%, daß alle Blowfly-Larven abgetötet wurden; 0% bedeutet, daß keine Blowfly-Larven abgetötet wurden.

Bei diesem Test zeigten beispielsweise die Verbindungen 6, 7, 8 und 11 der Herstellungsbeispiele eine hervorragende Wirkung.

Beispiel E

Schabentest

| | | |
|---|---|---|
| Testtiere: | Blattella germanica oder Periplaneta americana | |
| Lösungsmittel: | 35 Gewichtsteile<br>35 Gewichtsteile | Ethylenglykolmonomethylether<br>Nonylphenolpolyglykolether |

Zwecks Herstellung einer geeigneten Formulierung vermischt man drei Gewichtsteile Wirkstoff mit sieben Teilen des oben genannten Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (Ø 9,5 cm) pipettiert, die sich in Petrischalen entsprechender Größe befinden. Nach Trocknung der Filterscheiben werden 5 Testtiere bei B. germanica bzw. P. americana überführt und abgedeckt.

Nach 3 Tagen wird die Wirksamkeit der Wirkstoffzubereitung ermittelt. Die Wirksamkeit drückt man in % aus. Dabei bedeutet 100 %, daß alle Schaben abgetötet werden; 0 % bedeutet, daß keine Schaben abgetötet wurden.

Bei diesem Test zeigt beispielsweise Verbindung 6 der Herstellungsbeispieler eine hervorragende Wirkung bei 1000 ppm.

Beispiel F

Fliegentest

| Testtiere: | Musca domestica, Stamm WHO(N) | |
|---|---|---|
| Lösungsmittel: | 35 Gewichtsteile<br>35 Gewichtsteile | Ethylenglykolmonomethylether<br>Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wassere auf die jeweils gewünschte Konzentration.

2 ml dieser Wirkstoffzubereitung werden auf Filterpapierscheiben (Ø 9,5 cm) pipettiert, die sich in Petrischalen entsprechender Größe befinden. Nach Trocknung der Filterscheiben werden 25 Testtiere in die Petrischalen überführt und abgedeckt.

Nach 6 Stunden wird die Wirksamkeit der Wirkstoffzubereitung ermittelt. Die Wirksamkeit drückt man in % aus. Dabei bedeutet 100 %, daß alle Fliegen abgetötet werden; 0 % bedeutet, daß keine Fliegen abgetötet wurden.

Bei diesem Test zeigt beispielsweise Verbindung 7 der Herstellungsbeispieler eine hervorragende Wirkung bei 1000 ppm.

**Patentansprüche**

**1.** Substituierte 3,4-Hetarylpyrazoline der Formel (I)

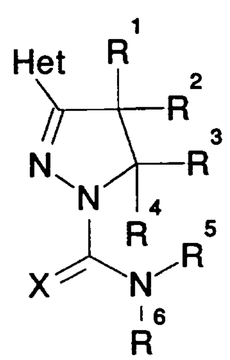

(I)

in welcher

$R^1$ für einen ungesättigten fünf- oder sechsgliedrigen, 1 bis 4 Stickstoffatome enthaltenden, gegebenenfalls substituierten und gegebenenfalls benzokondensierten Heterocyclus oder für einen gegebenenfalls substituierten Heterocyclus aus der Reihe

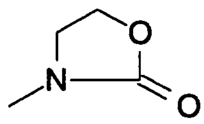,
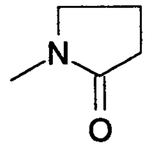,
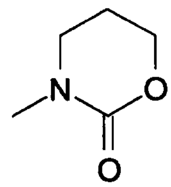,
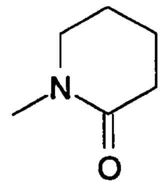,
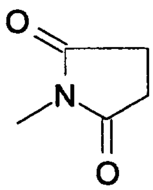,
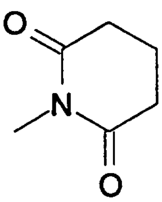

steht

$R^2$ für Wasserstoff, Alkyl, gegebenenfalls substituiertes Cycloalkyl, Halogenalkyl, Halogenalkylthio oder Alkoxycarbonyl steht,

$R^3$ für Wasserstoff oder Alkyl steht,

$R^4$ für Wasserstoff oder Alkyl steht,

$R^5$ für Wasserstoff, Alkyl, Phenyl oder Alkylthio steht,

$R^6$ für gegebenenfalls substituiertes Alkyl, gegebenenfalls substituiertes Cycloalkyl oder für den Rest

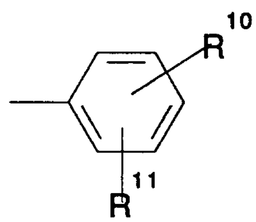

steht,

worin $R^{10}$ und $R^{11}$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

X für Sauerstoff oder Schwefel steht und

Het für einen gegebenenfalls substituierten und/oder gegebenenfalls anellierten Heterocyclus steht.

**2.** Verfahren zur Herstellung der substituierten 3,4-Hetaryl-pyrazoline der allgemeinern Formel (I) in welcher

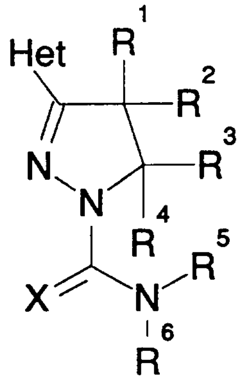

(I)

$R^1$ für einen ungesättigten fünf- oder sechsgliedrigen, 1 bis 4 Stickstoffatome enthaltenden, gegebenenfalls substituierten und gegebenenfalls benzokondensierten Heterocyclus oder für einen gegebenenfalls substituierten Heterocyclus aus der Reihe

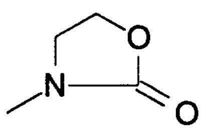 , 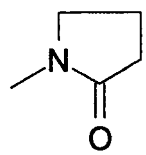 , 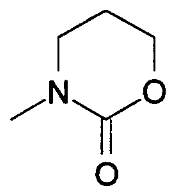 , 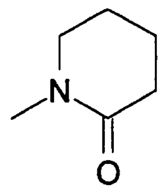 ,

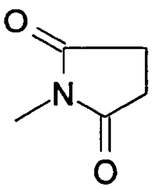 , 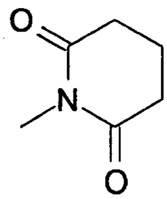

steht

$R^2$ für Wasserstoff, Alkyl, gegebenenfalls substituiertes Cycloalkyl, Halogenalkyl, Halogenalkylthio oder Alkoxycarbonyl steht,

$R^3$ für Wasserstoff oder Alkyl steht,

$R^4$ für Wasserstoff oder Alkyl steht,

$R^5$ für Wasserstoff, Alkyl, Phenyl oder Alkylthio steht,

$R^6$ für gegebenenfalls substituiertes Alkyl, gegebenenfalls substituiertes Cycloalkyl oder für den Rest

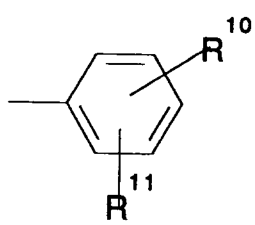

steht,

worin $R^{10}$ und $R^{11}$ gleich oder verschieden sein können und für Wasserstoff, Haloen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes Arylthio, Alkenyloxy, Alkinyl, Alkylhionyl, Alkylsulfonyl, Halogenalkylthionyl, Halogenalkylsulfonyl, Halogenlkxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

X für Sauerstoff oder Schwefel steht und

Het für einen gegebenenfalls substituierten und/oder gegebenenfalls anellierten Heteroyclus steht,

dadurch gekennzeichnet, daß man

(A) zum Erhalt von substituierte 3,4-Hetaryl-pyrazolinen der Formel (I), in welcher $R^5$ für Wasserstoff steht, Pyrazolinderivate der Formel (II)

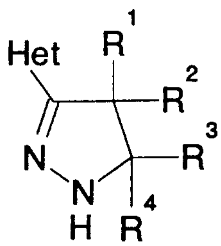

(II)

in welcher

$R^1$, $R^2$, $R^3$, $R^4$ und Het die oben angegebene Bedeutung besitzen,

mit Isocyanaten bzw. Isothiocyanaten der Formel (III)

$X=C=N-R^6$ (III)

in welcher

X und $R^6$ die oben angegebene Bedeutung haben,

gegebenenfalls in Gegenwart von Basen umsetzt oder wenn man

(B) Pyrazolinderivate der Formel (II)

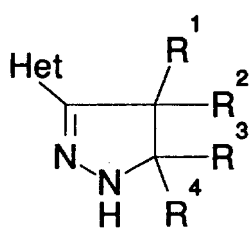

(II)

in welcher

$R^1$, $R^2$, $R^3$, $R^4$ und Het die oben angegebene Bedeutung haben,

mit Verbindungen der Formel (IV)

$R^6$-NH-CO-Cl (IV)

in welcher

$R^6$ die oben angegebene Bedeutung hat,

gegebenenfalls in Gegenwart eines Lösungsmittels und gegebenenfalls in Gegenwart von Basen umsetzt.

**3.** Substituierte 3,4-Hetaryl-pyrazoline gemäß Anspruch 1 der Formel (I), in welcher

$R^1$ für einen gegebenenfalls substituierten Pyrrolyl-, Pyrazolyl-, Imidazolyl-, 1,2,3-Triazolyl-, 1,2,4-Triazolyl-, Tetrazolyl-, Pyridinyl-, Pyridonyl-, Pyrimidinyl-, Pyrimidonyl-, Pyrazinyl-, Pyridazinyl-oder Triazinyl-Rest steht,

der weiterhin für einen gegebenenfalls substituierten und über Stickstoff gebunenen Azolinon-, Azolinthion- oder Azoliniminorest aus der Reihe

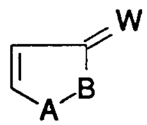 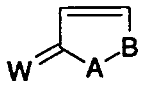 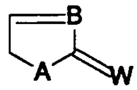 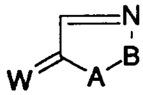 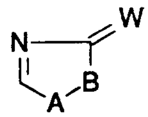 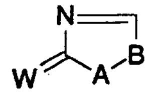

($R^1$-a) ($R^1$-b) ($R^1$-c) ($R^1$-d) ($R^1$-e) ($R^1$-f)

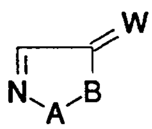 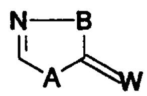 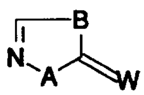 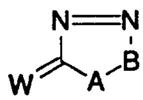 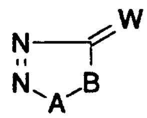 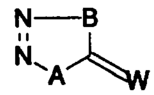

($R^1$-g) ($R^1$-h) ($R^1$-i) ($R^1$-k) ($R^1$-l) ($R^1$-m)

worin

eine der Gruppen A oder B für Stickstoff steht und jeweils die andere (A oder B) für Sauerstoff, Schwefel oder für die Gruppe - NAlkyl($C_1$-$C_4$-), für eine Methylengruppierung -$CH_2$-oder eine -CH-Gruppe steht,

W für Sauerstoff, Schwefel oder für die Gruppe -NAlkyl($C_1$-$C_4$-) steht, oder weiterhin für einen gegebenenfalls substituierten Heterocyclus aus der Reihe

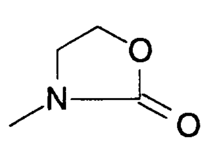 , 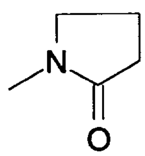 , 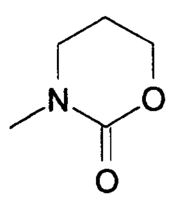 , 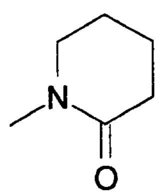 ,

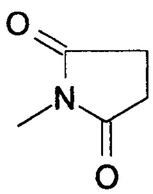 , 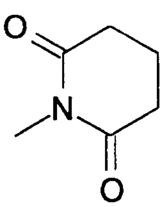

steht

worin für die oben aufgeführten Heterocyclen jeweils die folgenden Subtuenten infrage kommen:

Halogen, Cyano, Nitro, Hydroxy, Amino, Alkyl($C_1$-$C_6$), Alkoxy($C_1$-$C_6$), Alkyl($C_1$-$C_6$)-thio, Halogenalkyl($C_1$-$C_4$), Halogenalkoxy($C_1$-$C_4$), Halogenalkyl($C_1$-$C_4$)thio, Alkyl($C_1$-$C_6$)amino, Dialkyl($C_1$-$C_6$)amino, Dihalogenalkyl($C_1$-$C_4$)amino, Alkoxy($C_1$-$C_6$)carbonyl und gegebenenfalls durch Halogen, Alkyl($C_1$-$C_4$), Alkoxy($C_1$-$C_4$), Halogenalkyl($C_1$-$C_4$), Halogenalkoxy($C_1$-$C_4$) oder Halogenalkyl($C_1$-$C_4$)thio substituiertes Phenyl,

$R^2$ für Wasserstoff, Alkyl($C_1$-$C_6$), gegebenenfalls durch Halogen, Halogenalkyl($C_1$-$C_4$) substituiertes Cycloalkyl($C_3$-$C_7$); Halogenalkyl($C_1$-$C_4$)thio oder Alkoxy($C_1$-$C_6$)-carbonyl steht,

$R^3$ für Wasserstoff oder Alkyl($C_1$-$C_6$) steht,

$R^4$ für Wasserstoff oder Alkyl($C_1$-$C_6$) steht,

$R^5$ für Wasserstoff, Alkyl($C_1$-$C_6$), Phenyl oder Alkyl($C_1$-$C_4$)thio steht,

$R^6$ für gegebenenfalls durch Halogen, Halogenalkyl($C_1$-$C_4$), Halogenalkoxy($C_1$-$C_4$) sub-

stituiertes Alkyl($C_1$-$C_6$),
für gegebenenfalls durch Halogen, Halogenalkyl($C_1$-$C_4$),Halogenalkoxy($C_1$-$C_4$) substituiertes Cycloalkyl($C_3$-$C_7$) oder für den Rest

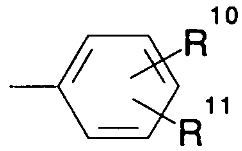

steht,
wobei

$R^{10}$ und $R^{11}$ gleich oder verschieden sein können und für Wasserstoff, Halogen, Alkyl($C_1$-$C_6$), Nitro, Cyano, Halogenalkyl($C_1$-$C_4$), Alkoxy($C_1$-$C_6$), Halogenalkoxy($C_1$-$C_4$), Alkyl($C_1$-$C_4$)thio, Halogenalkyl($C_1$-$C_4$)thio, gegebenenfalls durch Halogen, Halogenalkyl($C_1$-$C_4$), Alkoxy($C_1$-$C_4$), Alkyl($C_1$-$C_4$) substituiertes Phenoxy oder Phenylthio, gegebenenfalls durch Halogen, Alkoxy($C_1$-$C_4$),Halogenalkyl($C_1$-$C_4$) substituiertes Mono- oder Dialkylamino mit jeweils 1 bis 6 Kohlenstoffatomen im Alkylrest, gegebenenfalls durch Alkyl($C_1$-$C_4$), Alkoxy($C_1$-$C_4$), Halogen, Alkyl($C_1$-$C_4$)thio substituiertes Cycloalkyl($C_3$-$C_7$), Alkoxy($C_1$-$C_4$)carbonyl, Alkenyl($C_2$-$C_6$)oxy, Alkinyl($C_2$-$C_6$), Alkyl-($C_1$-$C_4$)thionyl, Alkyl($C_1$-$C_4$)sulfonyl, Halogenalkyl($C_1$-$C_4$)thionyl, Halogenalkyl($C_1$-$C_4$)sulfonyl, Halogenalkoxy($C_1$-$C_4$)-carbonyl stehen oder wobei

$R^{10}$ und $R^{11}$ zusammen für einen der folgenden bivalenten Reste stehen

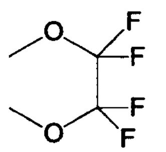
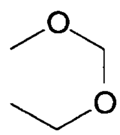
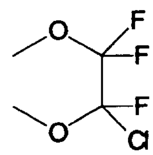
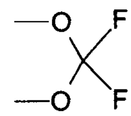

X für Sauerstoff oder Schwefel steht und

Het für einen unsubstituierten oder einfach bis mehrfach, gleich oder verschieden substituierten, gegebenenfalls benzoanellierten heteroaromatischen 5- oder 6-gliedrigen Ring, der gleiche oder verschiedene, ein oder mehrere Heteroatome wie Sauerstoff, Schwefel und Stickstoff enthält, wobei als Substituenten infrage kommen Alkyl, Halogen, Halogenalkyl, Alkoxy, Alkylthio, Halogenalkoxy, Halogenalkylthio, Alkoxycarbonyl, Halogenalkoxycarbonyl, gegebenenfalls substituiertes Aryloxy, gegebenenfalls substituiertes Arylthio, Alkenyloxy, Alkinyl, Alkylthionyl, Alkylsulfonyl, Halogenalkylthionyl, Halogenalkylsulfonyl, Nitro, Cyano oder wobei zwei benachbarte Positionen durch einen durch Halogen substituierten 3,4-Methylendioxyl- oder 3,4-Ethylendioxylrest miteinander verbunden sind.

**4.** Substituierte 3,4-Hetaryl-pyrazoline der Formel (I) gemäß Anspruch 1, in welcher

$R^1$ für einen gegebenenfalls substituierten Pyrrolyl-, Pyrazolyl-, Imidazolyl-, 1,2,3-Triazolyl-, Tetrazolyl-, Pyridinyl-, Pyridonyl-, Pyrimidinyl-, Pyrimidonyl-, Pyrazinyl-, Pyridazinyl-, 1,2,4-Triazolyl- oder Triazinyl-Rest steht oder

$R^1$ weiterhin für einen substituierten oder einfach oder zweifach, gleich oder verschieden substituierten und über Stickstoff gebundenen Azolinon, Azolinthion- oder Azoliniminrest aus der Reihe

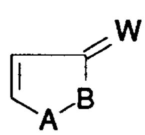
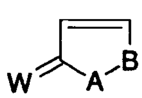
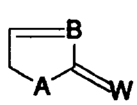
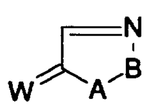
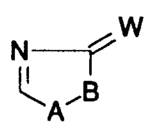
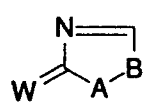

($R^1$-a) ($R^1$-b) ($R^1$-c) ($R^1$-d) ($R^1$-e) ($R^1$-f)

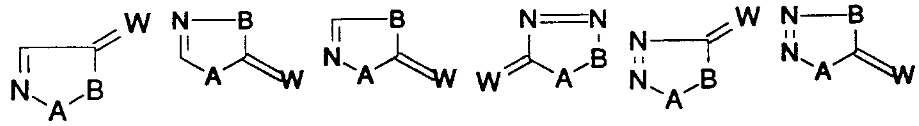

($R^1$-g) ($R^1$-h) ($R^1$-i) ($R^1$-k) ($R^1$-l) ($R^1$-m)

worin

eine der Gruppen A oder B für Stickstoff steht und jeweils die andere (A oder B) für Sauerstoff, Schwefel oder für die Gruppe - NAlkyl($C_1$-$C_4$-), für eine Methylengruppierung - $CH_2$- oder eine CH-Gruppe steht,

W für Sauerstoff, Schwefel oder für die Gruppe - NAlkyl($C_1$-$C_4$-) steht, oder

$R^1$ weiterhin für einen unsubstituierten oder einfach bis dreifach substituierten Heterocyclus aus der Reihe

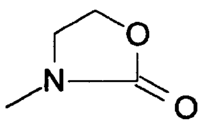 , 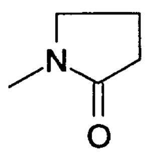 , 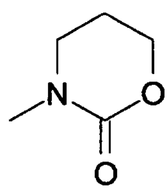 , 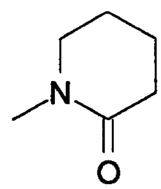 ,

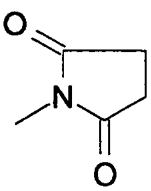 , 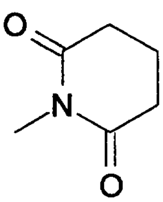

steht

worin für die oben aufgeführten Heterocyclen jeweils die folgenden Substituenten infrage kommen:

Fluor, Chlor, Brom, Iod, Cyano, Nitro, Hydroxy, Amino, Alkyl($C_1$-$C_4$), Alkoxy($C_1$-$C_4$), Alkyl($C_1$-$C_4$)thio, Halogenalkyl($C_1$-$C_2$), Halogenalkoxy-($C_1$-$C_2$), Halogenalkyl($C_1$-$C_2$)thio, Alkyl($C_1$-$C_3$)amino, Dialkyl($C_1$-$C_3$)-amino, Dihalogenalkyl($C_1$-$C_2$)amino, Alkoxy($C_1$-$C_4$)carbonyl und gegebenenfalls durch Fluor, Chlor, Brom, Methyl, Ethyl, Methoxy, Ethoxy, Halogenalkyl($C_1$-$C_2$), Halogenalkoxy($C_1$-$C_2$) oder Halogenalkyl-($C_1$-$C_2$)thio substituiertes Phenyl,

$R^2$ für Wasserstoff, Alkyl($C_1$-$C_4$), gegebenenfalls durch Fluor, Chlor, Brom, Halogenalkyl($C_1$-$C_3$) substituiertes Cycloalkyl($C_3$-$C_6$); Halogenalkyl($C_1$-$C_3$)thio oder Alkoxy($C_1$-$C_4$)carbonyl steht,

$R^3$ für Wasserstoff oder Alkyl($C_1$-$C_4$) steht,

$R^4$ für Wasserstoff oder Alkyl($C_1$-$C_4$) steht,

$R^5$ für Wasserstoff, Alkyl($C_1$-$C_4$), Phenyl oder Alkyl($C_1$-$C_3$)thio steht,

$R^6$ für gegebenenfalls durch Fluor, Chlor, Brom, Halogenalkyl($C_1$-$C_3$), Halogenalkoxy($C_1$-$C_3$) substituiertes Alkyl($C_1$-$C_4$), für gegebenenfalls durch Fluor, Chlor, Brom, Halogenalkyl($C_1$-$C_3$), Halogenalkoxy($C_1$-

$C_3$) substituiertes Cycloalkyl($C_3$-$C_6$) oder für den Rest

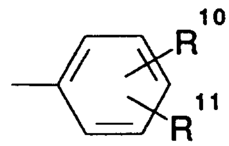

steht,
wobei

$R^{10}$ und $R^{11}$ gleich oder verschieden sein können und für Wasserstoff, Fluor, Chlor, Brom, Iod, Alkyl($C_1$-$C_4$), Nitro, Cyano, Halogenalkyl($C_1$-$C_3$), Alkoxy($C_1$-$C_4$), Halogenalkoxy($C_1$-$C_3$), Alkyl($C_1$-$C_3$)thio, Halogenalkyl-($C_1$-$C_3$)thio, gegebenenfalls durch Fluor, Chlor, Brom, Halogenalkyl-($C_1$-$C_3$), Alkoxy($C_1$-$C_3$), Alkyl($C_1$-$C_3$) substituiertes Phenoxy, gegebenenfalls durch Fluor, Chlor, Brom, Alkoxy($C_1$-$C_3$), Halogenalkyl($C_1$-$C_3$) substituiertes Mono-oder Dialkylamino mit jeweils 1 bis 4 Kohlenstoffatomen im Alkylrest, gegebenenfalls durch Alkyl($C_1$-$C_3$), Alkoxy-($C_1$-$C_3$), Fluor, Chlor, Brom, Alkyl($C_1$-$C_3$)thio substituiertes Cycloalkyl($C_3$-$C_6$) stehen oder wobei

$R^{10}$ und $R^{11}$ zusammen für einen der folgenden bivalenten Reste stehen

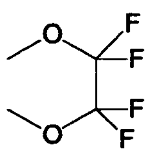 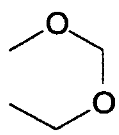 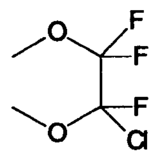 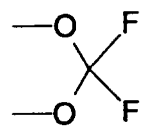

X für Sauerstoff oder Schwefel steht und

Het für einen unsubstituierten oder einfach bis dreifach, gleich oder verschieden substituierten heteroaromatischen 5- oder 6-gliedrigen Ring steht, der gleiche oder verschiedene, ein oder mehrere Heteroatome wie Sauerstoff, Schwefel oder Stickstoff enthält, wobei als Substituenten genannt seien:
Alkyl($C_1$-$C_4$), Fluor, Chlor, Brom, Halogenalkyl($C_1$-$C_4$), Alkoxy($C_1$-$C_4$), Alkyl($C_1$-$C_4$)thio, Halogenalkoxy($C_1$-$C_3$), Halogenalkyl($C_1$-$C_3$)thio, Alkoxy($C_1$-$C_3$)carbonyl, gegebenenfalls durch Fluor, Chlor, Brom, Alkyl($C_1$-$C_3$), Alkoxy($C_1$-$C_3$), Halogenalkyl($C_1$-$C_3$) substituiertes Phenoxy oder Phenylthio, Alkenyl($C_2$-$C_4$)oxy, Alkinyl($C_2$-$C_4$), Alkyl($C_1$-$C_3$)thionyl, Alkyl($C_1$-$C_3$)sulfonyl, Halogenalkyl($C_1$-$C_3$)thionyl, Halogenalkyl($C_1$-$C_3$)sulfonyl, Nitro, Cyano stehen oder wobei zwei benachbarte Positionen durch einen durch Fluor und/oder Chlor substituierten 3,4-Methylendioxy- oder 3,4-Ethylendioxyrest miteinander verbunden sind.

**5.** Substituierte 3,4-Hetaryl-pyrazoline der Formel (I) gemäß Anspruch 1, in welcher

$R^1$ für einen gegebenenfalls substituierten Pyrrolyl-, Pyrazolyl-, Imidazolyl-, 1,2,3-Triazolyl-, 1,2,4-Triazolyl-, Tetrazolyl-, Pyridinyl-, Pyridonyl-, Pyrimidinyl-, Pyrimidonyl-, Pyrazinyl-, Pyridazinyl-oder Triazinyl-Rest steht oder

$R^1$ weiterhin für einen unsubstituierten oder einfach oder zweifach, gleich oder verschieden substituierten und über Stickstoff gebundenen Azolinon, Azolinthion- oder Azoliniminrest aus der Reihe

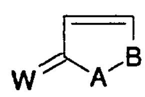 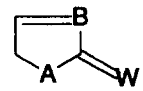 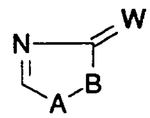 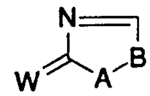

($R^1$-b) ($R^1$-c) ($R^1$-e) ($R^1$-f)

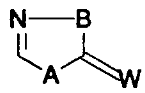 oder 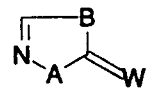

($R^1$-h) ($R^1$-i)

worin

eine der Gruppen A oder B für Stickstoff steht und jeweils die andere (A oder B) für Sauerstoff, Schwefel oder für die Gruppe - NAlkyl($C_1$-$C_4$-), für eine Methylengruppierung - $CH_2$- oder eine - CH-Gruppe steht,

W für Sauerstoff oder Schwefel steht, oder

$R^1$ weiterhin für einen unsubstituierten oder einfach bis dreifach substituierten Heterocyclus aus der Reihe

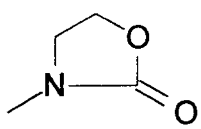 , 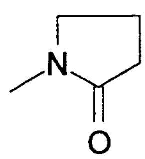 , 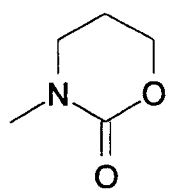 , 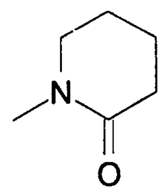 ,

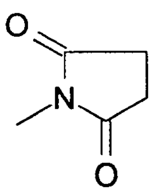 , 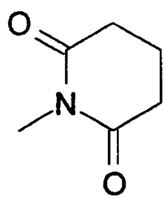

steht

wobei für die oben genannten Heterocyclen jeweils die folgenden Substituenten infrage kommen:

Fluor, Chlor, Brom, Iod, Cyano, Nitro, Hydroxy, Amino, Methyl, Ethyl, n-Propyl, i-Propyl, t-Butyl, Methoxy, Ethoxy, Methylthio, Ethylthio, Halogenalkyl($C_1$-$C_2$), Halogenalkoxy($C_1$-$C_2$), Halogenalkyl($C_1$-$C_2$)thio mit jeweils 1 bis 5 Fluor- und/oder Chloratomen, Methylamino, Dimethylamino, Methoxycarbonyl und gegebenenfalls durch Fluor, Chlor, Brom, Methyl, Ethyl, Methoxy, Ethoxy und Halogenalkoxy($C_1$-$C_2$) oder Halogenalkyl($C_1$-$C_2$)thio mit jeweils 1 bis 5 Fluor-und/oder Chloratomen substituiertes Phenyl,

$R^2$ für Wasserstoff, Methyl, Ethyl, n-Propyl, i-Propyl oder Alkoxy($C_1$-$C_2$)-carbonyl steht,

$R^3$ für Wasserstoff, Methyl, Ethyl, n-Propyl oder i-Propyl steht,

$R^4$ für Wasserstoff, Methyl, Ethyl oder n-Propyl steht,

$R^5$ für Wasserstoff, Methyl, Ethyl, n-Propyl, i-Propyl, Phenyl oder Alkyl-($C_1$-$C_2$)thio steht,

$R^6$ für gegebenenfalls durch Fluor, Chlor, Halogenalkyl($C_1$-$C_3$), Halogenalkoxy($C_1$-$C_3$) substituiertes Methyl, Ethyl, n-Propyl oder i-Propyl für gegebenenfalls durch Fluor, Chlor, Halogenalkyl($C_1$-$C_3$),

Halogenalkoxy($C_1$-$C_3$) substituiertes Cycloalkyl ($C_3$-$C_6$) oder für den Rest

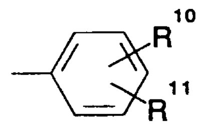

steht, wobei

$R^{10}$ und $R^{11}$ gleich oder verschieden sein können und für Wasserstoff, Fluor, Chlor, Brom, Iod, Methyl, Ethyl, n-Propyl, i-Propyl, tert-Butyl, Nitro, Cyano, Halogenalkyl($C_1$-$C_3$), Alkoxy($C_1$-$C_3$), Halogenalkoxy($C_1$-$C_3$), Alkyl($C_1$-$C_3$)thio, Halogenalkyl($C_1$-$C_3$)thio, gegebenenfalls durch Fluor, Chlor, Halogenalkyl($C_1$-$C_3$),Methoxy, Ethoxy, Methyl, Ethyl substituiertes Phenoxy, gegebenenfalls durch Fluor, Chlor, Methoxy, Ethoxy, Halogenalkyl($C_1$-$C_3$) substituiertes Mono- oder Dialkylamino mit jeweils 1 bis 3 Kohlenstoffatomen im Alkylrest, gegebenenfalls durch Methyl, Ethyl, Methoxy, Ethoxy, Fluor, Chlor, Alkyl($C_1$-$C_3$)thio substituiertes Cycloalkyl($C_3$-$C_6$) stehen oder wobei

$R^{10}$ und $R^{11}$ zusammen für einen der folgenden bivalenten Reste stehen

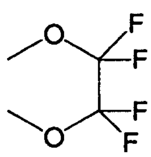 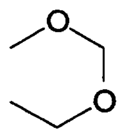 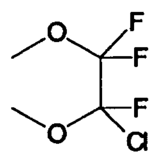 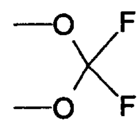

X für Sauerstoff oder Schwefel steht und

Het für einen jeweils unsubstituierten oder einfach bis dreifach, gleich oder verschieden substituierten Pryridyl-, Pyrazinyl-, Thiazolyl-, Pyrimidyl-, Pyridazinyl, Thienyl-, Furyl-, Oxazolyl- oder Pyrrolyl-Rest steht, wobei als Substituenten genannt seien:

Methyl, Ethyl, n-Propyl, i-Propyl, tert-Butyl, Fluor, Chlor,Brom, Iod, Halogenalkyl($C_1$-$C_3$), Methoxy, Ethoxy, n-Propyloxy, i-Propyloxy, Alkyl($C_1$-$C_3$)thio, Halogenalkoxy($C_1$-$C_3$), Halogenalkyl($C_1$-$C_3$)thio, Alkoxy($C_1$-$C_3$)carbonyl, gegebenenfalls durch Fluor, Chlor, Brom, Methyl, Ethyl, Methoxy, Ethoxy, Halogenalkyl($C_1$-$C_3$) substituiertes Phenoxy oder Phenylthio, Alkenyl($C_3$-$C_4$)oxy, Alkinyl($C_2$-$C_4$), Alkyl-($C_1$-$C_3$)thionyl, Alkyl($C_1$-$C_3$)sulfonyl, Halogenalkyl($C_1$-$C_3$)thionyl, Halogenalkyl($C_1$-$C_3$)sulfonyl, Nitro, Cyano stehen oder

wobei zwei benachbarte Positionen durch einen durch Fluor und/oder Chlor substituierten 3,4-Methylendioxy- oder 3,4-Ethylendioxyrest miteinander verbunden sind.

**6.** Schädlingsbekämpfungsmittel, gekennzeichnet durch einen Gehalt an mindestens einem substituierten 3,4-Hetaryl-pyrazolin der Formel (I) gemäß Anspruch 1.

**7.** Verwendung von substituiertem 3,4-Hetaryl-pyrazolin der Formel (I) gemäß Anspruch 1 zur Bekämpfung von Schädlingen.

**8.** Verfahren zur Bekämpfung von Schädlingen, dadurch gekennzeichnet, daß man substituierte 3,4-Hetaryl-pyrazoline der Formel (I) gemäß Anspruch 1 auf Schädlinge und/oder ihren Lebensraum einwirken läßt.

9. Verfahren zur Herstellung von Schädlingsbekämpfungsmitteln, dadurch gekennzeichnet, daß man substituierte 3,4-Hetaryl-pyrazoline der Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung
EP 93 11 5451

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,X | EP-A-0 466 408 (ROHM AND HAAS COMPANY) 15. Januar 1992<br>* Ansprüche 1,22 *<br>--- | 1,6 | C07D401/14<br>C07D409/14<br>A01N47/38 |
| P,X | EP-A-0 508 469 (DOWELANCO) 14. Oktober 1992<br>* Seite 23, Zeile 29 - Zeile 38; Ansprüche 1,6 *<br>--- | 1,6 | |
| D,A | GB-A-1 514 285 (N. V. PHILIPS GLOEILAMPENFABRIEKEN) 14. Juni 1978<br>* Ansprüche 1,19 *<br>----- | 1,6 | |
| | | | RECHERCHIERTE SACHGEBIETE (Int.Cl.5) |
| | | | C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 17. Januar 1994 | VOYIAZOGLOU, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument